# EUROPÄISCHE PATENTSCHRIFT

(11) **EP 1 965 791 B1**
(45) Veröffentlichungstag und Bekanntmachung des Hinweises auf die Patenterteilung: **23.07.2014**
(21) Anmeldenummer: 06828720.0
(22) Anmeldetag: 21.12.2006
(51) Int. Cl.: A61K 31/365, A61K 31/191, A61K 31/592, A61K 31/593, A61K 33/06, A61K 33/42

(54) **NEUE KOMPOSITIONEN GEGEN ALKYL-ACYL-GPC, SEINE DERIVATE UND PRODUKTE**
NOVEL COMPOSITIONS AGAINST ALKYL-ACYL-GPC, THE DERIVATIVES AND PRODUCTS THEREOF
NOUVELLES COMPOSITIONS CONTRE LE ALKYLE-ACYLE GPC, DES DERIVES DE CELUI-CI ET DES PRODUITS DE CELUI-CI

(30) Priorität: 27.12.2005 DE 102005624170
(43) Veröffentlichungstag der Anmeldung: 10.09.2008
(73) Patentinhaber: Korth, Ruth-Maria, 80639 München (DE)
(72) Erfinder: Korth, Ruth-Maria, 80639 München (DE)
(74) Vertreter: Beck, Alexander
(86) Internationale Anmeldenummer: PCT/DE2006/002294
(87) Internationale Veröffentlichungsnummer: WO 2007/073727

(56) Entgegenhaltungen:
- EP-A- 0 303 277
- EP-A2- 0 312 913
- WO-A-01/12208
- WO-A-2005/025587
- US-A- 5 605 927
- US-A- 5 852 052
- US-A- 5 895 785
- US-A1- 2002 127 287
- US-A1- 2004 101 578
- US-A1- 2004 180 105
- DATABASE BIOSIS [Online] BIOSCIENCES INFORMATION SERVICE, PHILADELPHIA, PA, US; März 2005 (2005-03), KORTH RUTH-MARIA: "Gender dyslipidemia and ether phospholipids" XP002430182 Database accession no. PREV200510322221 & FASEB JOURNAL, Bd. 19, Nr. 4, Suppl. S, Part 1, März 2005 (2005-03), Seite A109, EXPERIMENTAL BIOLOGY 2005 MEETING/35TH INTERNATIONAL CONGRESS OF PHYSIOLOGICAL SCIENCES; SAN DIEGO, CA, USA; MARCH 31 -APRIL 06, 2005 ISSN: 0892-6638
- DATABASE MEDLINE [Online] US NATIONAL LIBRARY OF MEDICINE (NLM), BETHESDA, MD, US; Dezember 1991 (1991-12), DOS SANTOS O F ET AL: "Effect of platelet-activating factor antagonist BN 52063 on the nephrotoxicity of cisplatin." XP002430183 Database accession no. NLM1819725 & LIPIDS DEC 1991, Bd. 26, Nr. 12, Dezember 1991 (1991-12), Seiten 1324-1328, ISSN: 0024-4201

## Beschreibung

Der Gegenstand der Erfindung ist in den Ansprüchen definiert.

### 1) Die neuen, erfindungsgemäßen Kompositionen

Die Kompositionen mit zumindest einem Prähormon, Peptid-Proteo-Hormon, Ginkgoloid, Mineral und Spurenelement sind neu, frisch und erfinderisch. Erstmals werden hormonellen und/oder diätetische Kompositionen zum hormonellen, prägenden Ausgleich verwendet. Erstmals wird ein Prähormon, Hormon aus der Mevalonat-, Cholecalciferol-Calcitriolgruppe und/oder der Peptid- Proteohormone mit Ginkgoloiden ergänzt. Erstmals werden Vorstufen von Hormonen (Prähormone) vorzugsweise aus der "Ergocalciferol- (D2), Cholecalciferolgruppe (D3) auch mit zumindest einem Spurenelement gemischt (z.B. mit Jodid, Selen, Zink). Die Erfindung betrifft also neue Mischungen für überraschende hormonelle Verwendungen gegen alkyl- und/oder acylartige Destabilisierungen von Zellen. Die neuen Kompositionen enthalten erstmals eine Mischung von Ginkgoloide, mit einem Mineral und/oder einem Spurenelementen.

Zudem überraschen neue zweite Verwendungen, die gegen Alkyl-Acyl-GPC, (AAGPC) seine Derivate (Alkyl-Lipide) und/oder Produkte (z.B. Acetylacyl-Coenzym A, CoA) gerichtet sind auch zum Schutz der Acetylcholin(re)synthese und der (zentralen) Hormone. Ginkgoloide hemmen hier die Bindung der Alkyl-Gruppe, die über LDL eine zelluläre Anreicherung vom AAGPC vermittelt. AAGPC ist die Muttersubstanz von Alkyl-Lipiden und der Acyl-, Cholingruppen und prägt so den Energiehaushalt aller Zellen. Erstmals wurde hier erarbeitet, wie AAGPC über die Bildung von Acetylacyl-Coenzym A (CoA) z.B. die Synthese von Mevalonaten steuert und/oder Acetyl-CoA hemmt und damit der Acetylcholin(re)synthese das Substrat entzieht und/oder die Abschwächung von Hormonen behindert. Die unerwarteten alkyl-, acylartigen Überempfindlichkeitssyndrome von Zellen, Organen, Drüsen, Geweben wurden klinisch behandelt. Auch die Zellorganeilen z.B. Cytosol, Microsomen, Lysosomen, Peroxysomen sind gestört, erschöpft. Um den Überempfindlichkeitssyndromen zu begegnen werden Ginkgoloide hier erstmals mit Acetylcysteinen, acetylierenden Substanzen kombiniert.

Die neuen Kompositionen mit zumindest einem Prähormon, Hormon und zumindest einem Mineral, Spurenelement mit Ginkgoloiden dienen der Herstellung von Mitteln für orale, diätetische und/oder lokale, kosmetische Verwendungen. Die (semi-)synthetischen, zubereiteten Komponenten sind antioxidativ stabilisiert und werden in nach außen einheitlicher, Form angeboten.

Die Alkyl-Lipide sind stabil und prägen Trägerproteine und Zellen im gesamten Organismus in hormonartiger Weise. Diese Alkoholmetabolite zeigen unerwartet frühe signifikante Schäden, denn freies Albumin sinkt und z.B. Hormone und/oder Lipide werden zunehmend schädlich, fast toxisch. Klinisch werden die alkyl-artigen Schäden z.B. Alkohol und/oder Nikotin durch die signifikanten Assoziationen der Albuminurie, Hämaturie, Diabetesneigung, Blutdruckerhöhungen gezeigt, die durch Hormone noch verstärkt werden. Dagegen gleichen die neuen Kompositionen aus und wirken hormonell, ganzheitlich und prägend. Zur Gesundheitsförderung wurden die alkyl-artigen Destabilisierungen nicht "forte...intens" antagonisiert, repariert sondern die klinischen Schäden wurden ausgleichend, prägend umgestellt, wobei auch eine verbesserte Lebensführung zur Minderung der Noxen führte. Sogar schwere alkoholische Nierenschäden wurden hier erfolgreich behandelt mit einem abstinenten Patienten und/oder der Ausheilung einer nikotinvermittelten Hämaturie, Diabetesneigung (Prädiabetes).

Die Ratio von Albumin zu Triglyceriden sinkt signifikant bei Alkoholproblemen oder Adipositas und zeigt damit den Abtransport von Albumin ins Gewebe an mit steigendem Blutdruck und Schäden vom Haut-Unterhaut-Bindegewebe. Beim Gesunden stellt Albumin 60% aller Plasmapeptide, wobei nur 40% vom Gesamtalbumin im Plasma verbleiben und nur freies Albumin schützt. Die Kompositionen schützen gegen unreines Albumin, das Alkyl-Lipide und Calcium ins Gewebe, Gehirn transportiert.

Pigmentstörungen werden als ein unerwartetes ganzheitliches Problem erkannt und lokal, hormonell ganzheitlich behandelt. Ginkgoloide mit Sonnenschutzmittel stärken alle Zellen und Gewebe. Nur antioxidativ stabilisierte Öle ohne ätherische Öle werden lokal verwendet (z.B. gereinigte Olivenöle, Sojaprodukte usw). Die zentralen melanotropen Peptidhormone werden hier moduliert, acetylierend abgeschwächt z.B. durch Acetylcysteine, Aminosäuren, Peptide, Multivitaminpräparate. Die neue Herstellungs-, Auswahl- und Darreichungsformen von Ginkgoloiden wurden im P34669914 erstmals offenbart, wobei diese neuen lokalen Kompositionen nach Bedarf angereichert werden z.B. mit Sonnenschutzmitteln, Mineralien, Folsäure, Proteoglycanen, Vitaminen Spurenelementen z.B. Selen und/oder mit Peptiden, Aminosäuren, antioxidativ stabilisierten Lipiden, Phospholipiden, Lecithinen, Ceramiden, ungesättigten Fettsäuren.

### 2) Die Komponenten:

### 2.1. Ausgleich des Mineralhaushalts:

Die hormonellen Kompositionen gleichen den gesamten Organismus aus. Erstmals wird ein schleichender Calciumverlust mit der Albuminurie erkannt und hormonell ausgeglichen. Die Kompositionen heilen die Albuminurie aus und begegnen damit dem schleichenden Knochenschwund über den Ausgleich der calciumabhängigen Parathormone (Peptidhormone) mit Phosphorbindung von Knochen und Zähnen.

Der Calciumverlust wird erstmals über die Albuminurie berechnet und ausgeglichen (0.8 mg Calcium pro mg Albumin pro Liter Urin). Der Bedarf von lichtabhängigen Hormonen, Prähormonen D2/D3 liegt bei 1000 IU pro Tag und der vom Calcium bei 600 mg/Tag. Der tägliche auch lipidsenkende, nervenschonende Bedarf vom Jod liegt bei 150 *µ*g/Tag. Wasser und Nahrung decken nicht immer den überraschend erhöhten Mineral- und Jodidbedarf, der hier ausgeglichen wird, z.B. brauchen Vegetarier auch Vitamin B12. Calcium (Calciumcarbonat den Calciumcitrat usw.), Phosphor (Phosphat usw) und Spurenelementen (Jod, Jodid, Zink, Selen) werden erstmals mit Gingkoloiden kombiniert.

Die Hormone werden ebenfalls nach Bedarf aus der Gruppe der Peptid-Proteohormone oder aus der Dihydroxy-Cholecalciferolgruppe, Calcitriole gewählt. Die Prähormone, Phytohormone aus der Gruppe der Dolichole, Mevalonate, Isoprenoide, Sqalene, Chinone, Farnesyle, Cholestane, Sterine werden ausgewählt, stabilisiert, angereichert. Diese bekannten Vorstufen und/oder lichtabhängige Prähormone D2 vorzugsweise aus der pflanzlichen Gruppe z.B. den Ergocalciferolen werden hier gemischt mit Prähormonen D3 vorzugsweise aus der tierischen Cholecalciferolgruppe (vgl. Harper's Biochemistry, 24th Edition, S.617). Antioxidativ stabilisierte Fischöle und/oder Pflanzenöle werden gemischt zur optimalen vorzugsweise öligen Lösung von Phytohormonen, Prähormonen, Vitaminen der Gruppe A,B,C,E, Lecithinen, ungesättigte Fettsäuren und Ceramide, Mineralien mit Calcium, Magnesium, Phosphor, Phosphate und Spurenelementen mit Jod, Selen, Zink. Die lichtgeschützten Pflanzenöle enthalten auch Phytosterine, Cholestan d.h. die Stammkohlenwasserstoffe für alle Sterine, Ubiquinone (Mitochondrienschutz), Squalene usw. als Vorstufen von Hormonen (Phytohormone, Prähormone). Freies z.B. bovines Albumin wird beigemischt auch zum Ausgleich vom Vitamin B12. Die (semi-)synthetischen, und/oder natürlichen Hormone, Spurenelemente, Gingkoloide werden mit entsprechend zubereiteten, antioxidativ stabilisierten natürlichen und/oder (semi-) synthetischen tierischen und pflanzlichen Produkten, Ölen gemischt. Beispielhaft wird hier ein antioxidativ stabilisiertes Hochseefisch-Ölkonzentrat vermischt mit Kürbiskernöl und (semi-)synthetischer Folsäure (z.B. 24 mg/ml, 1/1, vol/vol). Das Fischölprodukt enthält 35% Omega-3-Fettsäure, davon 18% Eicosapentaensäure (EPA) und 12 % Docohexaensäure und ist mit Vitamin E stabilisiert (10 mg/1g, 14.9I.E.) d.h. gegen die Oxidationen geschützt. Das Fischöl wird ausgewählt nach dem Anteil der Prähormone D2+D3 vorzugsweise 16 *µ*g/100g, Phosphor z.B. 300 mg/100g, Jod vorzugsweise 8.4 *µ*g pro g Fischöl. Fischöle enthalten auch ungesättigte Lecithine (Korth et al. Chem. Phys. Lipids 36, S. 209, 1985), die hier auch erfindungsgemäß stabilisiert werden z.B. mit Selen und/oder Vitamin E. Zubereitete, gereinigte, antioxidativ stabilisierte, ungesättigte Pflanzenöle dienen vorzugsweise als Träger und enthalten u.a. Prä(Phyto)hormone, Calcium, Phosphor, Phosphate, ungesättigte Fettsäuren, Polyphenole, Lecithine und/oder Jodid (z.B. 100 *µ*g).

### 2.2. Ginkgoloide:

Gingkoloide teilen sich klassische pharmakologische Regressionslinien zur Hemmung der Alkyl-Lipide. Die Auswahl von Ginkgoloiden z.B. der "Aufarbeitungen vbn Ginkgoliden als Gemisch" wurde hier der unerwarteten Klinik angepasst (adapted for medical use). Trotzdem bleibt der "chemically defined extract from Ginkgo biloba" der pharmakologische Maßstab (Korth et al., Eur. J. Pharmacol. 152, S. 101, 1988; EP 0312913), so dass hier BN 52021 als Leitsubstanz aller natürlichen Ginkgoloide und WEB2086 als Leitsubstanz aller synthetischen Ginkgoloide beispielhaft getestet werden. Die Ginkgoloide wurden hier erfolgreich mit neuen Methoden getestet, um sodann mit überraschenden Komponenten gemischt, ergänzt, verdünnt zu werden (z.B. 1/3, vol/vol) vorzugsweise als Trockensubstanz in Ölen oder hier mit alkoholfreien handelsüblichen Lösungen (2:1, v/v) wegen der häufigen Alkoholprobleme der Risikopersonen. In keinem der vielen vorpublizierten Herstellungsverfahren wurde auch nur die Idee in Erwägung gezogen (vgl. P 344669914), z.B. Ginkgoloide mit Jodid und/oder mit Sonnenschutzmitteln zu mischen. Hier werden erstmals Ginkgoloide z.B. mit Acetylcysteinen, Alkal-Erdalkali-Carbonat, Citrat (Zitronen-, Weinsäure) gemischt, so dass sich eine neue erfindungsgemäße Brausezusammensetzung der Komposition ergibt. Auch eine Kombination von Ginkgoloiden mit Spurenelementen ist neu und erfinderisch.

Zudem werden neue Herstellungs- und Auswahlverfahren offenbart für neue Ginkgolide, wobei P34669914 zitierend einbezogen ist. Mit neuen Screeningverfahren wurden neue Kompositionen erarbeitet, wobei erstmals fixierte Zellen, Zelllinien verwendet wurden. Auch Zellorganellen interagieren mit einer Vielzahl von Alkyl-, Acyl-Lipiden, aus dem AAGPC. Erstmals wird ein quantifizierter Vollblutextrakt "biologisches Paf" verwendet d.h. eine Mischung von allen Alkyl-Liganden, die mit Ginkgoloiden um gleiche Bindungsstellen wetteifern. Ginkgoloide werden so neu ausgewählt aus der Gruppe der Analogen und Homologen sowie natürlichen und/oder synthetischen Derivaten und/oder aus der großen Gruppe der bekannten Paf Antagonisten (Hwang, J. Lipid Med. 2, S.123-158, 1990). Die Ginkgoloide werden zudem neu ausgewählt aus der großen Gruppe der natürlichen Antagonisten einschließlich der Ginkgolide mit Mischungen davon/damit, der Ginkgolidderivate, der synthetischen Ginkgolide der Ginkgo-Extrakte (EGB71, EGB usw), der Phospholipide mit Paf Analogen, der synthetischen Substanzen aus der Gruppe der Triazolame, Brotizolame, Thienodiazepine, der Thioetherbenzodiazepine (z.B. BN 50739), der Etherbenzozepine, der Chlorophenylbenzo(dia)zepine oder aus Gruppe der Tetrahydrofurane, Cyclopentane und/oder aus der Gruppe der endogenen Modulatoren, der Proteohormon-, Peptidgruppe usw.

Die neuen Methoden mit fixierten Zellen dienen auch der Auswahl von neuen Ginkgoloiden, Mischungen davon/damit, synthetischen Ginkgoliden, zweckmäßigeren Ginkgo-Extrakten. EGB761 (EP 0312913) oder EGB usw, die zusätzlich antioxidativ stabilisiert werden mit Selen, Vitamin E, wobei erstmals ätherischen Öle ausgereinigt werden für lokale Verwendungen. Die Ginkgo-Kompositionen werden nicht als "forte..intens" Arzneimittel bereitgestellt, weil deren Nebenwirkungen von den Gesunden nicht erwünscht sind. Überdosierungen der Komponenten sind zu vermeiden z.B. Hypervitaminosen, Hypercalcinosen (Nierensteine), Blutungen.

### 2.2. Aufbaumittel bei Mangelsyndromen:

Aminosäure- und Glukoselösungen mit oxidative stabilisierten Lecithinen, Peptiden, (Phyto-)Hormone, Vitaminen eignen sich auch als Aufbaumittel, wobei freies Albumin beigemischt werden kann. Essstörungen, Alkoholprobleme, gestörter Alkoholabbau, alkylacylartigen Destabilisierungen ("AHA"-Syndrome) führen zu Appetitlosigkeit, Fehl-Mangelernährung, Fettunverträglichkeit und Albuminmangel. Milch- oder Sojaprodukte wurden mit Vitaminen und/oder Aminosäuren, Acetylcysteinen angereichert, um den Mangel von Albumin, Acetyl CoA, Mineralien usw. auszugleichen z.B. bei hepatorenalen Störungen, Dialysen, renalen Anämien. Milch wird hier als Aufbaumittel mit den Vitaminen der B-Gruppe angereichert und auch mit Früchten z.B. für Senioren. Bei schweren renalen Anämien können die körpereigenen Stammzellen mit Erythropoetin (Peptidhormon) stimuliert werden. Für Patienten in letzter Not, Hospize werden alle palliativen Mittel (Misteln usw.), Schmerzmittel ausgeschöpft.

### 2.3. Übergewichtssyndrome:

Die erfindungsgemäßen Kompositionen enthalten zubereitete, semisynthetische und/oder ausgereinigte Kompositionen. Sie ergänzen gesunde Nahrungsmittel eigener Wahl, ohne diese zu beanspruchen, und werden in nach außen einheitlicher Form angeboten. Die vorgenannten neuen Kompositionen werden noch ergänzt durch Jodid. Ballaststoffen wie z.B. Plantago ovata oder Weizenkleie mit Spurenelemente, Selenium werden den Kompositionen beigemischt.

Bei Übergewicht z.B. mit kritischer Schilddrüsensituation (strumig, hypothyreot, (post-) entzündlich usw) und/oder kritischen Lipidwerten werden die Kompositionen mit Jodid angereichert. Die unerwünschten Wirkungen von Hormonen, z.B. Thryroidhormonen werden hier erfindungsgemäß ausgeglichen. Mit den erfindungsgemäßen Kompositionen werden die "OMIH"-Übergewichtssyndrome behandelt, besonders der bei Alkohol- und/oder Nikotinkonsum oder nach Mangelzuständen, Ditäten, die alkylartigen Lipide ansteigen. Die alkylartige Destabilisierung der Zellen wird hier behandelt, wobei die Symptome Hämaturie, Diabetesneigung, und/oder Blutdruckerhöhung der Ausdruck, das Frühwarnsystem sind für eine gestörte glatte Gefäßmuskulatur und/oder gefährdete Drüsenzellen sowie Zell-, Gewebe-, Organschäden überhaupt. Die zellulären Störungen zeigen sich zuerst in der Albuminurie und/oder der Blutdruckerhöhung und später auch mit Diabetesneigung, im Blutbild, mit Störungen der Haut, des Knochens, des Gehirns usw. Wenn das freie Albumin beim "OMIH"-Syndrom verbraucht ist, schädigt Albumin synergistisch mit LDL über AAPC, dessen Derivate, Produkte (z.B. AcetylacylCoA) die Zellen im gesamten Organismus auch der Haut-, Unterhaut-, Fettgewebe, Drüsen-, Nervenzellen und Gehirn. Die neue Komposition heilte überraschend erfolgreich.

### 2.4. Hormonumstellungen, Hormone. Arzneimittel:

Der Ausgleich von Hormonen wird vorzugsweise über eine Hemmung der Produkte, Derivate vom AAGPC erreicht, um Schönheit, Energie, seelisch, geistiges Wohlbefinden zu fördern. Ein Ausgleich des Hormonhaushalts verbessert die kritische Lebensführung und umgekehrt. Erstmals bewirken Ginkgoloide auch einen hormonellen Ausgleich und tragen zur Ausheilung von frühen klinischen Schäden bei. Hormone werden besser vertragen, die sinnvoll sind gegen hormonelle Störungen z.B. grenzwertige Hypothyreosen, Klimakterium, Energieverlust, Schlafstörungen, Knochenschwund. Peptidhormone wie z.B. Thyroidhormone prägen bekanntlich die Genexpressionen, aber hier wird eine unerwünschte Diabetesneigung mit Hämaturie unter Thyroidhormonen beobachtet, die auch durch Reduktion des Nikotinkonsums erfolgreich behandelt wurde. Sodann heilten Hämaturie, Diabetesneigung aus, nachdem die Hämaturie der übergewichtigen Raucherin jahrelang eine alkyl-artige Destabilisierung der renalen Zellen angezeigt hatte, die durch Hormone eher verschlechtert wurde. Ein Überempfindlichkeitssyndrom der Zellen z.B. gegenüber Hormonen, Lipiden wurde bisher nicht erkannt, um hier erarbeitet und erstmals behandelt zu werden.

Für Schwangere (und ihre Kinder) mit Gefahr der Glukoseintoleranz, Hyperinsulinämie, Insulinresistenz, Kinderschwäche, Jugendfettsucht usw. werden vorzugsweise antioxidativ stabilisierte Pflanzen- und Fischöle zubereitet, die reich sind an ungesättigten und mehrfach ungesättigten Fettsäuren, Lecithinen, Jod, Folsäure, Selen, Jodid, Flavonglycosiden, Lecithinen, Vitaminen, Spurenelemente' und Mineralien. Milch-, Gemüse-Fischprodukte werden empfohlen. Hier werden erstmals Komponenten verwendet, um auch planzenta-gängiges Albumin zu reinigen (z.B. stabilisierte Fettsäuren, Lecithine, Selen). Den Schwangeren wird auch viel lichtbezogene Bewegung empfohlen, aber keine Paf Antagonisten, um die Wehentätigkeit nicht zu stören. Jodid, hydrophile Folsäure werden immer beigemischt für gebärfähige Frauen mit Kinderwunsch, bei Anämien und/oder beim alternden Gehirn, gegen Hypothyreosen oder Homocysteine. Die bekannt erfolgreiche gesundheitsfördernde Strategie in Folge für Schwangere wird im Prioritätsdokument P34669914 beschrieben:

Hydrophile Ginkgolide, die bisher als unwirksam galten, und/oder Lipide, antioxidativ stabilisierte Phospholipide, Peptide, synthetische Substanzen können die Alkyl-Glycerole, Alkyl-LPA, Lyso paf aus dem Albumin verdrängen wogegen die lipophilen Ginkgoloide und Jodid in/auf Zellen, Gehirn Lipoproteinen günstig wirken. Jodid gleicht die Schilddrüsenfunktion aus und senkt die Blutfette.

Die erfindungsgemäßen Kompositionen mit Ginkgoloiden und/oder Acetylcystein wirken ergänzend und ausgleichend auf alle Hormone und gegen Überempfindlichkeitssyndrome auch bei klassischen Hormonersatztherapien. Die Ginkgoloide begegnen einer unerwünschten, proliferativen Wirkung von Hormonen und/oder Acetylcystein moduliert über die Acetylierung deren Wirkung. Bei kritischer Lebensführung reichern sich Alkyl-Lipide, alkylartige Lipide und Acylgruppen an, wobei Alkyl-Acyl-GPC (AAGPC) die Zelle "alkylartig destabilisiert" über Derivate d.h. Alkyl-Lipide und Produkte d.h. AcetylacylCoA, mit Störungen z.B. der (Re-)Synthese vom Acetylcholin aus Cholin und Acetyl CoA. Acetylierende Produkte begegnen hier erstmals dem übererregten Ungleichgewicht und verhindern Aktivierung, Ausdifferenzierung (Apoptose), Erschöpfung (Degeneration) der Zellen. Die erschöpften Haut- und Nervengewebe reagieren ähnlich und lagern unerwünschte Stoffe ein. Alkyl-Acyl - GPC seine Derivate, Produkte destabiliseren die Zellen, die überempfindlich werden gegenüber Hormonen. Hormone, Phytohormone, Prähormone werden hier vorsichtiger, niedriger dosiert und erfindungsgemäß ergänzt durch Spurenelemente, Mineralien, Aminosäuren. Acetylcystein liefert Substrat, denn z.B. werden die pigmentbildenden Peptidhormone durch eine Acetylierung abgeschwächt und Acetyl CoA wird für die Acetylcholin(re)synthese bereitgestellt. Auch bei Entzündungen, Infektanfälligkeit sind Acetylcysteine und/oder Selen sinnvoll. Zur Förderung des jungen Gehirns und/oder bei genetischen Schwächen werden die neuen Kompositionen entsprechend angereichert besonders mit Acetylcysteinen, und/oder Jodid, Selen (z.B. bei Trisomien, Isoformen der Lipoproteine, Acetylhydrolasen, Alkoholreduktasen, Connexinen usw).

Die Kompositionen mit Ginkgoloiden begegnen in kausaler Weise den mentalen, neuronalen, zerebralen, anfallsartigen kardialen, zerebralen, epileptischen Störungen z.B. vom Gehirn oder vom Herzen und lindern Schmerzzustände. Die Nebenwirkungen von Hormonen, Medikamenten werden durch die Komposition vermindert. Arzneimittel, Peptidhormone wie Thyroid-Parathormone, Cholestan-Derivate, Antiepileptika oder Marcumar usw. erhöhen den Knochenraubbau, wogegen die Komposition in prägender, ausgleichender Weise wirkt.

### 2.5. Haut:

Die Hautschäden zeigen sehr früh die alkylartigen Zellschäden an, wobei auch die diätetischen Kompositionen die Faltenbildung vermindern und den hauteigenen Sonnenschutz verstärken. Sonnenschutzmittel und Gingkoloide werden hier erstmals kombiniert mit allen bekannten pflegenden lokalen Darreichungsformen. Lokale Zubereitungen mit freiem Albumin, antioxidativ stabilisierten Lecithinen, Ceramiden, ungesättigten Fettsäuren, Pflanzen-, Honigprodukten sind hier zur Pflege des Haut-, Bindegewebes geeignet. Die neue Komposition ist notwendig bei Essstörungen, Gewichtsproblemen, Hormon(problemen) Umstellungen, Fehlernährungen, bei Hormoneinnahme und/oder im Alter. Die Bindegewebsschwächen (Striae) und/oder Pigmentstörungen vorzugsweise Vitiligo, Altersflecken, Chloasmen, Melasmen. Melanosen, Lichtdermatosen werden hier erstmals ganzheitlich mit neuen perkutanen Mitteln und/oder durch Modulierung von (zentralen) Hormonen geheilt. Eine kosmetische, diätetische, pflegende Komposition gegen Pigmentstörungen enthält ein Ginkgoloid gegen Alkyl-Acyl-GPC, dessen Derivate, Produkte mit zumindest einem Sonnenschutzmittel oder eine acetylierende Substanz um die ähnlichen Zellen der Haut, des Haut-, Bindegewebes und des Nervengewebes lokal und zentral zu schützen. Eine Kombination von spezifischen und unspezifischen Antagonisten schützt zudem gegen Alkyl-Lipide.

Die erfindungsgemäßen Kompositionen sind neu und überraschend frisch, denn erstmals werden Ginkgoloide mit Spurenelementen und/oder lichtabhängige Prähormone mit Lecithinen und/oder Ginkgoloide mit Sonnenschutzmitteln gemischt.

### 3) Die unerwarteten klinischen Problemstellungen und Pathomechanismen:

Bisher konnten die überraschenden Problemstellungen nicht erkannt werden und waren nicht nahe liegend. Erstmals im Prioritätsdokument P34669914 werden die unerwarteten klinischen Risikokonstellationen beschrieben auch mit Verlaufstudien, so das die neue Kompositionen erstmals den bisher unerkannten Problemen begegnet nämlich der kritischen Lebensführung mit (1) Nikotinkonsum, (2) Überempfindlichkeit gegenüber Hormonen, Lipiden und (3) Hämaturie, Diabetesneigung und/oder den (4) Alkoholproblemen mit signifikanten assoziierten Schäden (5) d.h. frühen Nierenschäden, Albuminurie, Hämaturie, Proteinurie, (6) überempfindlicher glatter Gefäßmuskulatur, Blutdruckerhöhung und/oder der Albuminurie mit (7) dem Calciumverlust als Beginn des Knochenabbaus, des Zahnverfalls und (8) dem gestörten Verhältnis von Albumin zu Lipiden, das den schädigenden Albuminabstransport mit Gewebe-, Hautschäden anzeigt (Alb/Trig) borderline, "OMIH"-Syndrom). Die frühen Schäden auch von gebärfähigen Frauen mit Gewichtsproblemen (z.B. 36±14 Jahre) und signifikante Geschlechtsunterschiede führten zu hormonellen, diätetischen, pflegenden Kompositionen, die (10) unerwartet erfolgreich eine seelische, affektive, sensitive Ausgeglichenheit erreichten. Eine verbesserte Lebensführung d.h. (11) weniger Risikofaktoren gleichen hier Gewichts-, Energie-, Schönheits-, Ernährungs-, Alkohol-, Nikotinproblemen aus und führten (12) zur unerwarteten klinischen Heilung.

Die langjährigen klinischen Studien (Bayerische Landesärztekammer, Ethik-Kommission Nr. 02088) zeigten die überraschenden Frühwarnsysteme. Erstmals wurde die fettig alkylartige Destabilisierung mit "OMIH"-Zellen als Problem des gesamten Organismus erkannt und mit den erfindungsgemäßen Kompositionen heilten sogar Nierenschäden und Diabetesneigung aus. Die auslösenden Schadstoffe wurden notwendigerweise reduziert, weil die stabilen Alkyl-Lipide in Peroxysomen aus fettigen Alkoholen gebildet werden. Nikotin vermittelt zudem eine alkylartige Wirkung durch Oxidierung von Lipiden. Die Zellen destabilisieren also durch Alkohol, Nikotin und werden überempfindlich, wobei diese Risikokombinationen entscheiden. AAGPC und dessen Derivate (Alkyl-Lipiden), Produkte (Acetylacyl CoA) reichern sich in den Zellen an und stellen den Energiehaushalt um, indem z.B., über Acetylacyl CoA die Mevalonate gebildet werden d.h. vermehrt Cholesterolvorstufen in allen Zellen, Zellorganellen, im Zytosol, endoplasmatischen Retikulum, Zellkern (DNA) usw., so dass die Zellen verfetten. Die erfindungsgemäßen Kompositionen hemmt die Bildung vom Alkyl-Acyl-Produkten. Das "OMIH"-Syndrom ist ein Grenzwert-(Borderline) Syndrom mit fettig alkyl-acyl-artig destabilisierten Zellen bei seelischer Unausgeglichenheit. Diese Zellen sind hier überempfindlich gegenüber allen differenzierenden Substanzen (Hormone, Lipide usw), wobei erschöpfte renale Zellen die Hämaturie erklären, erschöpfte Drüsenzellen die Diabetesneigung, gestörte Hautzellen die Pigmentstörungen. Klinisch wurden Blutdruckerhöhung, Prädiabetes, Albuminurie, Hämaturie, Proteinurie ("OMIH"-Syndrom) gebessert. Zum Beispiel zeigten Personen mit Alkoholproblemen einen erhöhten, Blutdruck, der hier von 145±22/89±13 mmHg auf 123±12/82±10 mmHg gesenkt wurde. Sie zeigten auch eine frühe Albuminurie (≥20 mg/l), die von 50 % auf 20% gesenkt wurde in den unpublizierten Daten der ersten Risikogruppe (AHA, n=5, ±1 S.D).

Die Kompositionen behandeln ganzheitlich, langfristig und niedriger dosiert und mindern so die vorhersagbaren Risiken und Schäden d.h. vaskuläre, neurovaskuläre, neuromuskuläre, zerebrale Störungen, wobei Alkohol und Nikotin reduziert werden. Die Kompositionen begegnen den nun voraussagbaren degenerativen, zerebralen Leiden, z.B. Schlaganfallrisiken bevor irreversible Leistungsstörungen erkennbar wurden z.B. Organ-, Hirn- und/oder Durchblutungsstörungen. Die zentral wirksamen Verwendungen schützen vor anfallsweisen Leiden, Kopfschmerzen, Krämpfen, Epilepsie. Unter nichtsteroidalen Antirheumatika nehmen dagegen Hypertonie, Blutungsneigung zu und Kognition nimmt ab, so dass die Kompositionen diese Mittel hier im Hinblick auf Schmerzanfälle und/oder Überempfindlichkeiten weitgehend ersetzten.

Blutdruckerhöhung und der Prädiabetes zeigen klinisch die unerwarteten Schäden von differenzierten Zellen wie z.B. der glatten Gefäßmuskelzellen von kleinen Gefäße (Arteriolen, Kapillaren, Venolen) oder der insulinproduzierenden Drüsenzellen vom Pankreas. Erstmals werden Prädiabetes und Hämaturie nachhaltig geheilt, wobei die Kompositionen mit Hormonen, Gingkoloiden und zumindest einem Mineral, Spurenelement auch die großen Gefäße schützen, deren Arterienwand über Vasi vasorum ernährt werden.

Der Albumin-Calcium-Transport ins Gewebe wird ganzheitlich, früh behandelt, um alle Gewebe auch das Gehirn gegen die schädigenden Liganden vom Albumin zu schützen. Venen und Lymphgefäße werden zudem gestärkt, so dass weniger schädigende Albumin-Liganden ins Gewebe austreten. Nur freies (reines) Albumin schützt durch Aufnahme von Alkyl-Lipiden, aber beim "OMIH"-Syndrom gibt es kaum noch freies Albumin. Albuminurie mit Blutdruckerhöhung zeigen die Gefahr, die durch LDL und kritische Lebensführung über die Alkyl-, alkylartigen Lipide gesteigert wird. Der signifikante, frühe Blutdruckanstieg beim "OMIH-Syndrom" wird durch die erfindungsgemäße Komposition gebessert. Der Energiehaushalt (Mitochondrien, Peroxysomen) mit neurovaskulären Regulationen (endotheliale Perizyten, glatte Muskulatur) wurde hier pflegend ausgeglichen, wobei die langzeitig erfolgreiche Therapie eine hormonelle, genetische, regenerierende Umstellung belegt.

Das "OMIH-Syndrom" wurde von der Fachwelt als eigenständiges Syndrom anerkannt, wobei die früh schädigenden Grenzwerte überraschten ("so early !!"). Diese unerwartete Grenzwertsyndrome zeigte sich klinisch als "overweight, mixed hyerlipidemia and/or intolerance to glucose (borderline) hypertension and critical morning urine samples". Ganz besonders schädigend waren dabei erhöhtes. LA-paf im LDL bei Nikotin- und Alkoholkonsum, die signifikant assoziiert waren mit Blutdruckerhöhung, Diabetesneigung und Albuminurie (Tabelle 2). Die Therapie ist also der Anfang einer gesunden Lebensmitte und kein dürftiges Ende nach manifestem Diabetes mellitus, Hypertonie, Fettsucht d.h. Metabolischem Syndrom mit sekundärer Demenz usw.. Die Abwehr, die Gesundheit, das Wohlbefinden, die Schönheit, die Lebensfreude, die Ausgeglichenheit, die seelische, mentale Gesundheit werden rechtzeitig, ausgleichend gefördert in einer ganzheitlichen Strategie, die Bewegung und Licht einbezieht. Der Ausgleich von Blutdruckerhöhungen, Diabetesneigung Hämaturie und Hautstörungen begegnet dem Alter ("happy aging").

Den Gefäß-, Herzleiden, neurovaskuläre Störungen, (sekundären) Demenzen, Gewebe, Organschwächen, Alterungs-, Schönheits-, Abwehr-Stoffwechselproblemen wird kausal begegnet. Ein seelisch, geistig, mentaler, zerebraler, endokrinologischer Ausgleich wird erreicht. Gegen Anfallsleiden vorzugsweise gegen alkoholbedingte, kardiale, zerebrale, seelisch, geistige, neuronale, mentale, gegen Schmerzattacken und/oder gegen psychotische und/oder paranoide Phasen wird geschützt. Sogar die schweren Alkoholschäden vom "Rosenkavalier" heilten aus und/oder die Nikotinschäden vom "Saturn".

Eine andere Variante der Erfindung besteht im Ausgleich von Hormon-Medikamentenwirkungen. Hormone schützen gegen vorzeitige Alterung und bessern die kritische Lebensführung, aber vermitteln auch Knochenabbau (Parat-Thyroid-Steroidhormone) und/oder Melanosen und können vaskuläre Degenerationen und mentale Krisen (Depressionen, Psychosen) auslösen, so dass die erfindungsgemäßen Kompositionen diesen Hormon-Überempfindlchkeiten kausal begegnen müssen besonders bei den vorgenannten Risikopersonen. Zudem lindern die Kompositionen kognitive Störungen, die durch Medikamente z.B. Psychopharmaka eher verstärkt werden (z.B. durch trikzyklische Antidepressiva).

Über die Albuminurie wird erstmals der frühe, schleichende Calciumverlust angezeigt und beseitigt, indem auch der Calcium- und Phosphathaushalt aufbauend ausgeglichen wird. Alle Zellen werden geschützt auch Knochen-, Zahn, Muskel-, Nerven-, und/oder Organschwächen. Der Calciumabtransport aus Knochen-, Zahnsubstanz wird gehemmt, wobei z.B. die Intimaverkalkungen oft parallel zum Knochencalciumabbau verläuft. Die bekannten degenerativen, calcium-, amyloidabhängigen Nervenzellschäden sind besonders dramatisch bei/mit den AAGPC-vorgeschädigten Nervenzellen. Zudem wird Fettgewebe reduziert, gereinigt und damit auch dessen hypertensive und entzündliche Substanzen vermindert (z.B. CRP usw.). Hypertonie, Diabetesneigung und auch Amyloide steigen bei chronischen Entzündungen an, wobei die neuen Kompositionen auch im Alter, bei alimentären, genetischen Risiken für degenerative Erkrankungen und/oder bei Überempfindlichkeitssyndromen außerordentlich wichtig sind. Die neuen Kompositionen binden Calcium und Phosphor im Knochen, Hormone und Ginkgoloide gleichen aus und Acetylcysteine schwächen Hormone und liefern Substrat für das Acetyl-CoA. In dieser Weise wird das Gehirn kausal und nachhaltig geschützt.

Die Albuminurie, Proteinuria, Hämaturie im ersten Morgenurin zeigt auch ein Risiko zur Karzinogese an, sowie genetische, neoplastische Schäden oder späte renale, urologische Probleme, denen früh begegnet wird. Nephropathien, Neoplasien, Karzinogenese sind behandelbar durch die frühe verbesserte Diagnostik der ersten Morgenurine (Proteinurie, Hämaturie, Albuminurie, Proteinprofile, Urinmikroskopie, Antikörper, Proteome, Blutchemie, Gene, Genkarten usw.).

Für jüngere Risikopersonen werden niedrigere Konzentrationen vorzugsweise zum Schutz des jungen Gehirns bereitgestellt, wobei auch die Entwicklung des frühkindlichen Gehirns gefördert wird besonders bei genetischer Gefährdung. Acetylcystein wird hier überraschend verwendet zum Ausgleich vom Acetyl-CoA z.B. bei genetischen Isoformen der Acetylhydrolasen. Acetylcysteine wirken gegen Entzündungen und fördern das kindliche Gehirn z.B. während der Schwangerschaft. Die erfindungsgemäßen Kompositionen ergänzen hier eine gesunde Ernährung z.B. bei entzündlichen Magen-Darmerkrankungen und/oder Appetitlosigkeit nach/bei Mangel, Bedarf (compositions adapted for medical use).

Besonders Frauen haben einen hohen Bedarf an den gesundheitsfördernden Kompositionen mit lichtabhängigen Hormonen gegen den Knochenschwund. Die neuen Kompositionen werden also für ein geschlechtsbezogenes Gesamtkonzept entwickelt im besondern Hinblick die klinischen Unterschiede zwischen Frauen und Männern. Neue Herstellungsverfahren sind im P3466914 spezifiziert und hier zitierend einbezogen für die lokalen Kompositionen mit Hormonen. Männer berichten hier öfter über Alkoholprobleme und reagieren eher mit systolischen, diastolischen Blutdruckerhöhungen und niedrigem HDL. Frauen neigen eher zu Übergewicht und kritischen LDL-Werten. Sie rauchen oft gegen ihr Übergewicht und/oder zum Ausgleich von seelischen, sozialen, mentalen Problemen. Männer und Frauen mit kritischer Lebensführung Alkohol-, Nikotin-, Gewichtsproblemen zeigen ein signifikant erniedrigtes Verhältnis von Albumin zu Triglyceriden "Alb/Trig", wobei das gestörte Verhältnis mit der erfindungsgemäßen Komposition gebessert wurde. Hier werden die plasmatischen, extravaskulären, zellulären Träger (Alb/Trig usw), gesäubert und der Hormonhaushalt normalisiert. Die erfindungsgemäßen Kompositionen verbessern die Lebensführung und auch darüber das Übergewicht, die gemischte Hyperlipidämie, Blutdruckerhöhungen. Die überempfindliche glatte Gefäßmuskulatur von Arteriolen, Venolen kann sich erholen. Glukoseintoleranz mit Albuminurie, Proteinurie, Hämaturie d.h. das "OMIH-Syndrom" heilen aus.

Die erfindungsgemäßen Kompositionen schützten hier ganzheitlich vorzugsweise alle Zellen des gesamten Organismus und durchsaften Knorpel, Knochen, Muskeln, Gewebe, Sehnen mit reinerem freien Albumin. Auch Risikopersonen werden geschützt vorzugsweise Frauen, Jugendlichen, Abhängige und/oder alternden, invalide, Personen und/oder Personen mit Gewichts-, Stoffwechsel-, Alkohol- und/oder Nikotinproblemen ("AHA-Syndrome", "OHMI"-Syndrom), denn frühe und späte Störungen heilten aus. Der Aufbau von Körper und Seele besserte auch die Lebensführung.

### 4) Die Haut-, Schönheitsprobleme:

Neue Kompositionen für die Haut wurden zum Schutz von Hautzellen (Keratinozyten und Melanozyten) besonders gegen Pigmentstörungen erarbeitet. Eine Anreicherung von Alkyl-Lipiden (AAGPC/LA-paf) im gesamten Organismus bewirkt, Pigmentstörungen der Haut, denen Sonnenschutzmittel begegnen. Lokal werden reinere Kompositionen, Komponenten ohne ätherische Öle verwendet besonders bei kritischer Lebensführung, im Alter und/oder bei Hormonumstellungen, in der Schwangerschaft usw. Der Lichtschutz der Haut wird gestärkt durch den seelisch, geistig, mentalen Ausgleich, der auch zur besseren Lebensführung führt mit weniger Gewichts-, Sucht- und Gewebeproblemen. Diese Probleme führten Herstellung einer risikorientierten kosmetischen Strategie mit den erfindungsgemäßen dietätischen und/oder lokalen Kompositionen. Die unerwarteten Pigmentstörungen der Haut durch ätherische Öle im EGB 761 führten zur erfindungsgemäßen Komposition mit Ginkgoloiden und Sonnenschutzmitteln.

Die lokalen und diätetischen Kompositionen sind aufeinander abgestimmt. Lichtabhängige Hormone, Vorhormone sind nötig beim sehr intensiven Sonnenschutz durch Externa oder Kleidung. Sonnenschutz ist ansonsten erwünscht. Bei besonderen Risiken, Frauen, Fehlernährungen, Kulturgrenzen, erhöhtem Bedarf, Alter, genetischen Risiken, unordentlicher Lebensführung sind die lichtabhängigen Hormone auszugleichen und/oder bei sehr intensivem Sonnenschutz. Die melanotropen Hypophysenhormone werden durch Acetylierungen geschwächt.

Sonnenschutzmittel werden erfindungsgemäß den lokalen Kompositionen beigefügt, wenn lokale Darreichungsformen mit Ginkgoloiden die Haut pflegen sollen. Entsprechende lokale Kompositionen von Sonnenschutzmitteln, Ginkgoloiden, antioxidative stabilisierten Lecithinen und Ceramiden schützten die Haut besonders vor Pigmentschäden z.B. durch ätherische Öle. Die erfindungsgemäßen Kompositionen als orale und/oder kosmetische Darreichungsformen gleichen auch hormonell aus und stärken die Zellen des gesamten Organismus auch der Haut, Kopfhaut, des Fettgewebes, des Gehirns, der Organe, Gewebe überhaupt.

### 5) Die Galeniken:

Die erfindungsgemäßen Kompositionen werden mit isolierten, aufgearbeiteten, zubereiteten und/oder (semi-)synthetischen Komponenten hergestellt, wobei alle Komponenten antioxidativ stabilisiert werden im Hinblick auf das vorgenannte klinische Risikoprofil. Analoge, homologe, synthetische Derivate sind z.B. aus Pflanzen, Tieren, Mirkoorganismen, Pilzen und Produkte von Zelllinien und Bakterien.

Die Galeniken vom P34669914 werden zitierend einbezogen. Die oralen und/oder lokalen, perkutanen Darreichungsformen werden bevorzugt, aber subkutane, intramuskuläre, transdermale, intravenöse, trans-, intranasale, rektale, inhalative usw. Formen sind möglich. Tabletten, Dragees, Kapseln, Elixiere, Lösungen, Suspensionen, Gase, Sirups usw. können mit allen entsprechenden, nicht-toxischen Adjuvantien hergestellt werden.

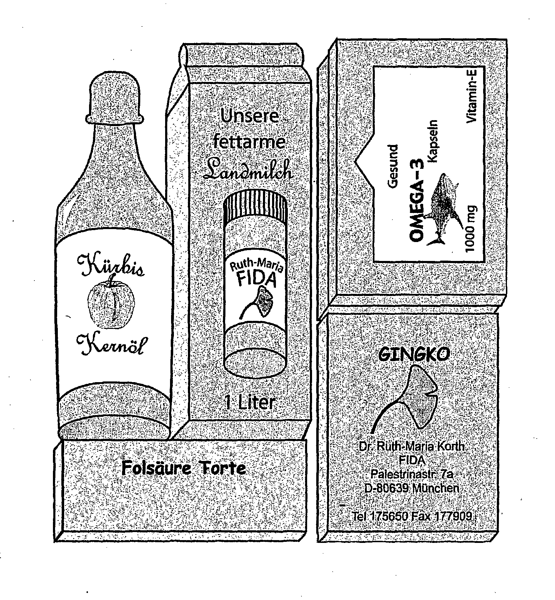

Beispielhaft werden hier die antioxidativ stabilisierten Kompositionen in einem sterilen Behälter gemischt, der mit Inhaltsangabe, Herstellungsdatum, Verfallsdatum (ungefähr 6 Monate) und mit einer der Marken der Anmelderin in nach außen einheitlich Form bereitgestellt werden (Figur 1 , z.B. EU-Marke 001089622). Der Behälter wird in einem lichtgeschützten zweiten Behälter mitgegeben. Die Komposition ergänzt hier z.B. fettarme Milch (1:9, vol/vol), ohne diese zu beanspruchen.

### 5.1. Ergänzungsmittel:

Die erfindungsgemäßen Kompositionen wirken hormonell und ergänzen Hormone, Nahrungsmittel, Arzneimittel. Thyroidhormone werden mit Jodid im Handel angeboten, wobei Jodid hier erstmals mit erfindungsgemäßen Kompositionen angereichert wird. Die Hormonersatztherapien werden im Handel als oral-dermal-vaginal-rektale Kombipackungen (Kits) angeboten, wobei hier erstmals die erfindungsgemäßen Kompositionen zusammen mit (Phyto-) Hormonen bereitgestellt werden. Im Handel sind Kombipackungen von Arzneimitteln gegen/beim Knochenabbau (z.B. Biphosphonate) mit zweizeitiger Verwendung von Vitamin D und Calcium, wobei die erfindungsgemäße Komposition hier erstmals mit Ginkgoloiden, Spurenelementen, Prähormonen zubereitet wird. Die Calciumverbindungen werden als klassische Brausemittel auch mit Ginkgoloiden zubereitet.

Die Kompositionen wirken hier erstmals synergistisch mit Hochdruckmittel(n) zum Beispiel mit ß-Blockern und Aldosteronantagonisten, so dass Kombipackungen (Kit) sinnvoll sind (z.B. auch mit Angiotensinantagonisten) oder wie zu Beginn der Behandlung auch mit Diuretika. Kombipackungen bieten sich hier besonders an zur Ergänzung von lipidsenkenden Substanzen (z.B. Statinen, Fibraten, Benzodiazepin-, Benzozepinderivaten usw) und/oder allen Diabetesmittel(n) (z.b.Cannaboidrezeptorantagonisten, Insulinsensitizer). Kombi-Packungen (Kits) eignen sich, um die Kompositionen gleichzeitig oder mehrzeitig lokal, perkutan und/oder oral in nach außen einheitlicher Form anzubieten. Zudem können Risikopersonen ihre Kombipackungen bedarfsweise anpassen entsprechend ihrer Nahrung nach Wahl, ihren Licht-, Bewegungs-, Lebenskrisen und ihren Schönheitsproblemen. Kombinationen sind hier besser geeignet als hochdosierte Präparate z.B. "Ginkgo Spezialextrakte ..forte intens" wegen der erhöhten Blutungsgefahr der Risikopersonen (z.B. von Hypertonikern, Leberzirrhosen, Nephrosen).

### 5.2. Neue Herstellungsverfahren:

Erfindungsgemäß werden hier reinere Wirksubstanzen erstmals mit öligen Zubereitungen hergestell die antioxidativ stabilisiert und entprechend ausgereinigt sind. Erstmals wird die Fettlöslichkeit der Komponenten genützt. Die neuen Herstellungs- und Auswahlverfahren vom Prioritätsdokument P34669914 werden hier zitierend einbezogen. Zum Beispiel bestimmen die lipophilen t-Butyl-Gruppen die Wirksamkeit von Ginkgoliden und damit von Ginkgo biloba Extrakten, so dass Trilaktone mit nur einer Butylgruppe (Bilobalid usw.) hier keine Ginkgoloide sein können. Jede zusätzliche polare Gruppen oder sogar eine Öffnung nur eines Lactonrings schränken die Wirksamkeit von Gingkoliden dramatisch ein (R. Korth, Eur. J. Pharmacol. 152, S. 101, 1988). Die peripheren Zellen werden vorzugsweise mit peripheren Benzodiazepinderivaten gegen Alkyl-Lipide geschützt, weil die entsprechenden pharmakologischen Regressionslinien geteilt werden.

Die antioxidativ stabilisierten, angereicherten Trägeröle werden in lichtgeschützten Ampullen (2-10 ml) angeboten oder mit Kapseln (z.B. 1-2 ml). Die Kapselmaterialien erlauben dabei retardierte, nanotechnologische, zweizeitige oder kontinuierlich Freisetzungen der Kompositionen und/oder Komponenten. Erfindungsgemäß werden zubereitete lichtgeschützte Pflanzenöle (z.B. Kürbiskernöle, Olivenöl, Soja) mit antioxidativ stabilisierten Fischölen gemischt, die sodann problemlos mit Ginkgoloiden, Ballaststoffen, Jodid angereichert werden z.B. gegen Übergewicht. Ausgewählte stabilisierte Pflanzenöle und Fischöle werden im Hinblick auf ihre optimalen Inhaltsstoffe ausgewählt, so dass Aminosäuren Peptide, Phytohormone, Hormone, Prähormone, Chinone, Enzyme (z.B. Biotine), Proteine, Peptide, Sterine, Mineralien z.B. Calcium, Phosphor, Magnesium und Vitamin A,B,C,E,K, Jodid, Selen in gelöster Form enthalten sind. Lecithine und ungesättigte Fettsäuren werden dabei zusätzlich antioxidativ stabilisiert (Selen,Vitamin E), so dass Antioxidantien in den Kompositionen im Überschuss enthalten sind. Diese Öle nehmen lipophile und hydrophile Ginkgoloide auf und bieten keine weiteren Probleme des Geschmacks oder Lösungsmittelprobleme.

Die diätetischen vorzugsweise öligen Kompositionen enthalten Phospholipide, die bekanntlich polare und unpolare Substanzen solubilisieren (vgl. Lipoproteine), aber die Phospholipide müssen zusätzlich antioxidativ stabilisiert werden, damit keine Kopfschmerzen, Krämpfe (Migräne, Epilepsien, Arrythmien usw) auftreten. Lipophile Komponenten durchdringen Membranen und werden in Zellen, Zellorganellen, Geweben, im Gehirn angereichert mit zellulären, zentralen Wirkungen. Ginkgoloide als spezifische, und antioxidativ stabilisierte Lecithine als unspezifische Alkyl-Antagonisten werden mit den neuen Methoden ausgewählt und umfassen eine große Gruppe von hydrophilen und/oder lipophilen, synthetischen, semi-synthetischen Substanzen. Die zusätzliche antioxidative Stabilisierung ist bedeutend, weil mit den öligen Trägern gezielt höhere Wirkspiegel aufgebaut werden im gesamten Organismus, auch im Gehirn. Mit unspezifischen und spezifischen Alkyl-Antagonisten wurde hier eine neue ölige Komposition klinisch erarbeitet, die beispielhaft erfolgreich, regenerierend heilten ohne Nebenwirkungen.

### 5.3. Brausemittel:

Besonders die Kombination mit Acetylcysteinen und/oder Calciumcarbonat, Calciumcitrat eignen sich für Brausemittel. Klassische Verfahren werden hier bevorzugt, weil Ginkgolide entgegen andersseitiger, evtl. profitorientierter Behauptungen nicht abgeschwächt werden durch moderate pH-Schwankungen. Brausemittel (Pulver, Granulate, Tabletten) werden erst kurz vor Einnahme mit Wasser versetzt, um sodann problemlos eingenommen zu werden. Die erfindungsgemäßen Kompositionen enthalten stabile Ginkgoloide und sind zudem sorgfältig antioxidativ stabilisiert(z.B. mit Selen, Vitamin E). Der Verlust von instabilen Trilaktonen d.h. vom Bilobalid durch die Herstellung von klassischen Brausemitteln ist hier ausdrücklich erwünscht (Oschmann DE 19509856, Anm. 17.3.1995). Die freien klassischen Verfahren, mit denen seit Jahrenzehnten Brausemittel hergestellt werden z.B. mit Acetylcysteinen und/oder Calciumpräparaten sind geeignet für die erfindungsgemäßen Kompositionen mit den "Aufarbeitungen von Ginkgoliden als Gemisch" von der Anmelderin (R.-M. Korth EP 0312913; Schmidt et al., Die Pharmazie, Februar 1990, Vol. 45, S. 89-101).

### 5.3. Pflegemittel:

Die lokalen Darreichungsformen werden ohne ätherische Substanzen oder Duftmittel hergestellt, um Pigmentsstörungen zu vermeiden. Ätherische Öle werden ausgereinigt, reinere Substanzen werden verwendet und nur antioxidativ stabilsierte Lecithine, Ceramide, ungesättigten Fettsäuren werden verwendet für die erfindungsgemäßen lokalen Darreichungsformen. Mit diesen hochwertigen Trägern werden Gingkoloide auch mit Sonnenschutzmitteln in nach außen einheitlicher Form angeboten.

Die erfindungsgemäßen lokalen, perkutanen, kosmetischen Kompositionen werden gleichzeitig oder mehrzeitig lokal und/oder oral z.B. als Kombipackungen angeboten.. Kurpackungen, angereicherte Haut-, Kopfhaut-, Zahnmittel, Pflaster, Gele, Creme-Lotionen können verwendet werden sowie alle bekannten galenischen Mittel.

Bei Bindegewebsproblemen werden z.B. ölige Substanzen einmassiert, die sodann mit einer zweiten fettreicheren Creme verstärkt werden für eine Langzeitwirkung z.B. bei Schwangerschaft oder gegen Übergewicht z.B. als typisches Hormon-, Alkohol, Altersproblem. Die Pflaster, Gele usw. z.B bei Hormon-, Schmerzmittelgabe erreichen ähnliche Langzeiteffekte.

Bei Schönheitsproblemen und/oder Bindegewebsproblemen fördern Packungen, Auflagen, Masken die kosmetische Wirkung durch die vorgenannte Art, intertriginöse Zone. Fettige Zubereitungen verbessern die Tiefenwirkung besonders zweizeitig und mit durchblutungsfördernden, bindegewebsstärkenden physikalischen Anwendungen (Wechselduschen usw.).

Bei Kopfhautproblemen wirken z.B. schonende nächtliche Kuren mit Ginkgo-Ölen, Hormonen, Vitaminen besonders der A,B,C-Reihe, Kieselerde, Weizenkleie mit Selen, die einmassiert werden. Diese Kuren begegnen auch endokrinologischen Kopfhautproblemen mit natürlichen, synthetischen, semi-synthetischen Hormonen und wirken z.B. gegen die weibliche Stirnglatze zusammen mit den erfindungsgemäßen oralen Kompositionen z.B. als Kombipackung. Morgens wird die Kopfhaut vorzugsweise mit lecithinhaltigen Produkten gereinigt, die auch Ginkgoloide enthalten können. Auch bei Männem wirken diese Kuren sehr gut auch gegen Kopfjucken und Schuppen. Gegen dermatologische Haut-, Kopfhautprobleme vorzugsweise mit Juckreiz wirken gekühlte Gele und/oder fettreiche Kompositionen mit Ginkgoloiden und Vitaminen der B-Reihe besonders gut. Diese Gele, Fette werden zweizeitig auch für die Nacht einmassiert. So wird die Haut ausgeglichen, antientzündlich gepflegt und besser durchblutet. Bei Kindern und Erwachsenen mit Neurodermitis, Sonnenbrand, Insektenstichen usw. wurde der quälende Reiz, Schmerz, gelindert und die Haut ausgleichend gepflegt

### 5.4. Liposomen:

Besonders die Liposomen (z.B. R.Korth, EP 0648 488, Anm. 14.9.1994) eignen sich für die erfindungsgemäßen Kompositionen. Ginkgoloide, Prähormone, Hormone, Mineralien und Spurenelement werden mit Liposomen gleichmäßig aufgenommen und können z.B. gleichzeitig intrazellulär wirken. Liposomen transportieren verschieden lösliche Komponenten, auch die hydrophile Folsäure. Schwierige Wirkorte werden erreicht für eine intra-, extrazerebral, intradermal, perkutane, extradermale, gleichzeitige und/oder mehrzeitige Wirkung im durchbluteten und durchsafteten Gewebe mit pflegender, reinigender Wirkung auch auf Lipide, Peptide, Proteine, Lipoproteine usw.

Als Galenik sind Liposomen freier Stand der Technik und vorpublizierte Mischungen und Darreichungsformen können hier erfindungsgemäß ergänzt werden, da die Kompositionen neu sind und überraschend. Die erfindungsgemäßen Kompositionen zur Pflege der Haut, Kopfhaut schützten erstmals mit Ginkgoloide und Sonnenschutzmitteln gleichzeitig oder, zweizeitig und in allen bekannten Galeniken auch mit Liposomen. Zudem sind antioxidativ stabilisierte Lecithine als unspezifische Alkyl-Antagonisten erwünscht und antioxidativ stabilisierte langkettige Ceramiden pflegen (z.B. aus Soja). Die Ausreinigung der ätherischen Ölen ist sehr wichtig, um Pigmentstörungen der Haut zu vermeiden, die durch Hormone, Alkyl-Lipide und/oder ätherisch Öle verstärkt werden und/oder durch die schädigende auch unspezifische Aufnahme von Alkyl-Lipiden (Pigmentstörungen, Vitiligo, Melanosen usw.). Die ergänzenden oralen Kompositionen hemmen z.B. über Acetylierungen der zentralen melanotropen Peptidhormone.

### 5.5. Mittel gegen Pigmentstörungen:

Hochwertige stabilisierte kosmetische Träger ohne ätherische Öle, Duftstoffe werden ausgewählt. Galeniken werden an den speziellen Haut-, Anwendungs- und/oder Hormontyp angepasst. Eine unerwartete pigmentstörende Wirkung wurden z.B. mit den Ginkgo-Spezialextrakten (EGB, EGB761) im bezeugten Selbstversuch beobachtet, wobei die neue lokale Komposition diese Pigmentstörungen ausglichen. Das Prioritätsdokument P3466914 offenbart erstmals die Kombination von Ginkgoloide, mit Sonnenschutzmitteln in nach außen einheitlicher Form. Die Trägeröle, Ginkgoloide und/oder Gingko-Extrakte wurden erstmals von ätherischen Ölen befreit.

Ginkgo-Spezialextrakt "forte...intens usw", die zu Pigmentstörungen führten, wurden gemieden. Nahrungsprodukte, Kräutermischungen, "Arzneimittel überhaupt" für innen und außen eignen sich nicht für/gegen die neuen Überempfindlichkeitssyndrome. Symptomatische Therapien mit "forte..intens für innen und außen" ohne präzise Wirkmechanismen blieben hinter den überraschend präzisen, ganzheitlichen Therapieverfahren zurück.

Niedriger konzentrierte (verdünnte), hochwertige, reinere Komponenten werden hier in nach außen einheitlicher Form angeboten, um die Überempfindlichkeitssyndrome auszugleichen zum Wohle des Hautgewebes und des Hormonhaushalts. Lipophile und hydrophile, spezifische und unspezifische Antagonisten begegnen hier erstmals wirkungsvoll den nachhaltigen Schäden durch die Alkyl-Lipide im gesamten Organismus.

### 6) Unerwartete Pathomechanismen und neue Methoden:

Die Figur 1 zeigt die erfindungsgemäße Komposition, die gegen unerwartete Risikokonstellationen erarbeitet wurde, die Komposition wird markenrechtlich gekennzeichnet FIDA^{R}. Sie besteht aus 3ml Kürbiskernöl, 2ml Fischöl, wobei Vitamin E hier antioxidativ stabilisiert, 1ml Folsäure und 3ml Ginkgolidlösung. Die erfindungsgemäße Komposition ergänzt Nahrungsmittel (2 ml/Tag) hier z.B. mit einer fettarmen Milch (unbeansprucht).

Folgend wird experimentell gezeigt, wie Alkyl-Acyl-GPC mit seinen Alkyl-Acyl-Cholingruppen angereichert wird und dabei allosterisch wirkt. Diese alkylacylartigen Destabiliserungen werden nicht durch Apoproteine (ApoB) und nicht durch Cholesterol und/oder Triglyceride (VLDL) vermittelt, sondern über einen allosterischen Alkyl-Effekt, die hier mit fixierten Zellen gezeigt wird. Die genauen Methoden sind erstmals im P346609914 ausführlich beschrieben und sind hier zitierend einbezogen.

### 5.1.Fettige alkylartige Zelldestabilisierung von differenzierten Zellen:

Die fettige Anreicherung von AAGPC wird untersucht, wobei AAGPC und Derivate hier gehemmt werden durch Ginkgoloide und freies Albumin. Menschliche Thrombozyten werden untersucht, behandelt als Modell für differenzierte Zellen z.B. für Nerven-, Herz-, Haut-, Knochenzellen, glatte und gestreifte Muskulator, Organ- Drüsenzellen wie z.B. Betazellen vom Pankreas, Hypophyse usw. Die Zellen beim "OMIH"-Zellen waren in vivo negativ geprägt, denn sie binden mehr Alkyl-Lipide und bilden mehr Alkyl-Acyl-GPC mit Derivaten, Produkten. Zellen, Zellorganellen (Peroxysomen, Motochondrien, DNA) destabilisieren, wobei die erfindungsgemäßen Kompositionen hier die klinischen Schäden heilte.

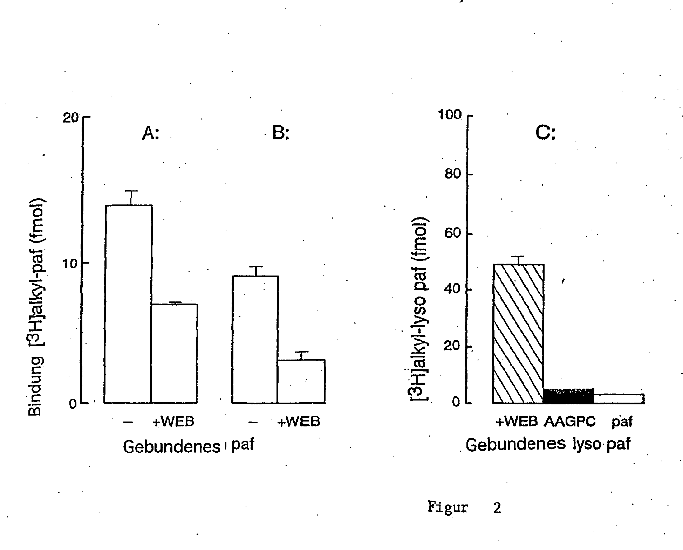

Die Figur 2 zeigt eine erhöhte unspezifische Bindung von Alkyl-Lipiden (hier z.B. [³H]alkyl-paf) an gewaschene, aspirinierte Thrombozyten beim "OMIH"-Syndrom (A). Diese gewaschenen, aspirinierten "OHMI"-Zellen binden mehr [³H]alkyl-paf im Vergleich zu den normolipidämischen Thrombozyten (B), die hier kaum [³H]alkyl-lyso paf aufnehmen und deshalb kaum AAGPC und LA-paf bilden (C). Die Frauen mit "OMIH"-Syndrom zeigten Übergewicht (34±11 Jahre, BMI: 25±5 kg/m²), erhöhte LDL- (167±15 mg/dl, HDL: 76±41 mg/dl) und Triglycerid-Werte (206±27 mg/dl) einen grenzwertigen Blutdruck (125±13/88±10 mmHg) und eine gestörte Glukosetoleranz (1Std: 170±61 mg/dl). Drei von den vier Frauen tranken Alkohol und zwei zeigten Hämaturien und/oder Proteinurien.

Ginkgoloide mit freiem bovinen Albumin (BSA) hemmten hier die Bindung vom [³H]alkyl-paf auch an die dreimal gewaschenen "OMIH"-Thrombozyten (Figur 2 A+B). Die hohe unspezifische Bindung vom [³H]alkyl-paf zeigte die alkylabhängige Prägung (A), die auch mit den nachgenannten allosterischen Alkyl-Effekten gestützt wird. Normolipidämische Thrombozyten nehmen kaum [³H]alkyl-paf oder [³H]alkyl-lyso paf auf und bilden kaum AAGPC und LA-paf in der Gegenwart von Gingkoloiden und freiem Albumin (Figur 2C).

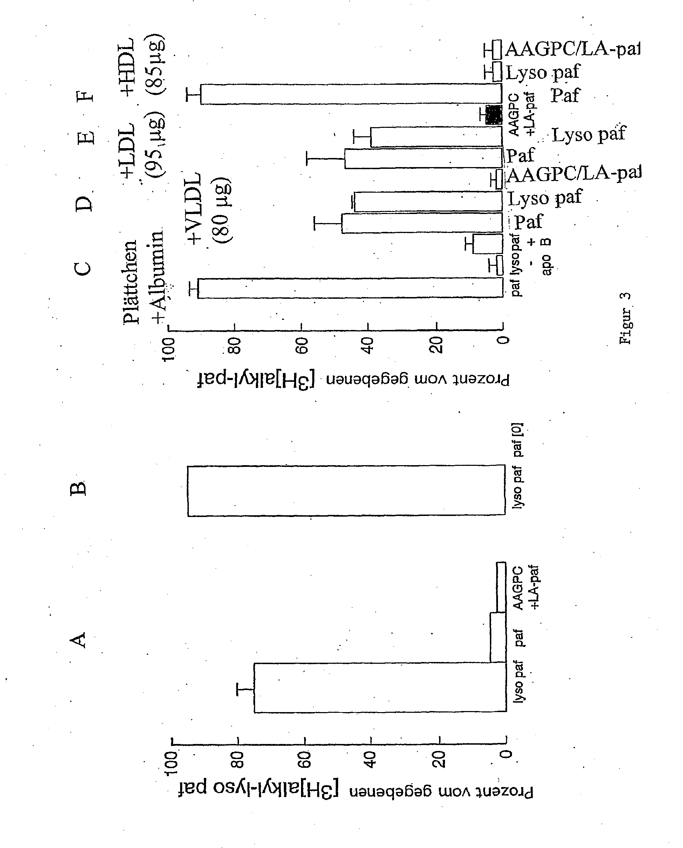

Die Figur 3 zeigt den Mechanismus der AAGPC-Produktion, wobei LA-paf im LDL die differenzierten Zellen aufnahmebereit macht (Figur 3E versus 3A-C). Nur LDL aber nicht ApoB und nicht VLDL überwinden den Albuminschutz (Figur 3E versus 3D). Apo B oder VLDL bilden Lyso paf ohne zelluläre Aufnahme (Figur 3 C+D), während freies, fettarmes Albumin oder HDL das [³H]alkyl-lyso paf und/oder [³H]alkyl-paf internalisieren, stabilisieren mit oder ohne Zellen (Figur 3A-C, F). In Anwesenheit (A+C) oder Abwesenheit von gewaschenen Thrombozyten (B) wird kaum AAGPC mit Derivaten, Produkten gebildet. Die Acetylhydrolasen vom LDL, vom ApoB und VLDL bilden Lyso paf, aber die Aufnahme vom Lyso paf wird nur durch LDL getriggert.

Die Figuren 2 und 3 beinhalten die klare therapeutische Anweisung, das schädigende AAGPC mit seinen Derivaten und Produkten zu senken (Figur 2 A-C, Figur 3C+F), um einer LDL-vermittelten Aufnahmebereitschaft von Zellen zu begegnen. Alkyl-Acyl-sn-glycero-3-Phosphocholine (AAGPC) reichern sich sonst in Zellen an und ist bekanntlich das Substrat für Lipasen, Hydrolasen, Transferasen usw mit bekannter Bildung, Freisetzung von Alkyl-Glycerolen, Alkyl-Phosphoglycerole (LPA), Alkyl-Phospholipiden, Alkyl-Ethanolaminen, Alkyl-Lipiden, Alkyl-Cannaboiden. Auch die Acyl-Produkte vom AAGPC werden hier erfindungsgemäß gehemmt gegen neue Überempfindlichkeitssyndrome.

### 5.2. Fettige alkylartige Zelldestabilisierung von undifferenzierten Zellen

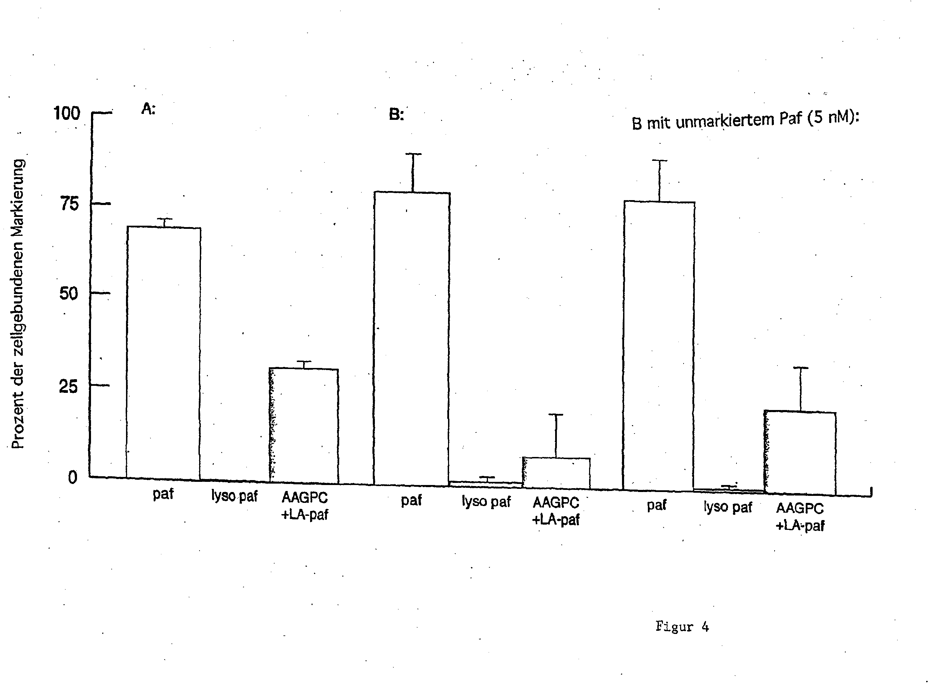

Die Figur 4 zeigt, die gesättigte, unspezifische Aufnahme von Alkyl-Lipiden durch undifferenzierte Zellen. Die undifferenzierteren gewaschenen Blutleukozyten nehmen hier [³H]alkyl paf über [³H]alkyl lyso paf auf und bilden zelluläre Alkyl-Lipide (AAGPC+LA-paf) in gesättigter Weise (A: 0.65 nM vs. B: 6.5 nM), wobei unmarkiertes Paf nicht verdrängt wird (B, 5nM). Damit ist zumindest ein unspezifisches Transportprotein (z.B. Acetylhydrolase) auf undifferenzierten Zellen gezeigt. Die weniger differenzierten Zellen werden hier modellhaft untersucht für Monozyten Endothelzellen, Gliazellen, Knorpelzellen, Fettzellen, körpereigene Stammzellen usw.. Mit ihren Acetylhydrolasen auf Plasmamembranen entsorgen also die leukozytäre Zellen die Alkyl-Lipide, aber die Zellen präaktivieren, differenzieren und emigrieren. Unspezifische Antagonisten z.B. freies Albumin schützten gegen die Alkyl-Liganden und wetteifern mit den Zellen um die Alkyl-Lipide. Die fettige Zellschädigung im gesamten Organismus wird hier klinisch geheilt. Erfindungsgemäß wird auch experimentell weniger AAGPC gebildet durch Ginkgoloide mit freiem Albumin, um der fettig alkylacylartige Destabilisierung von Zellen grundlegend zu begegnen.

### 5.3. Bindung von Alkyl-Lipiden an fixierte Zellen:

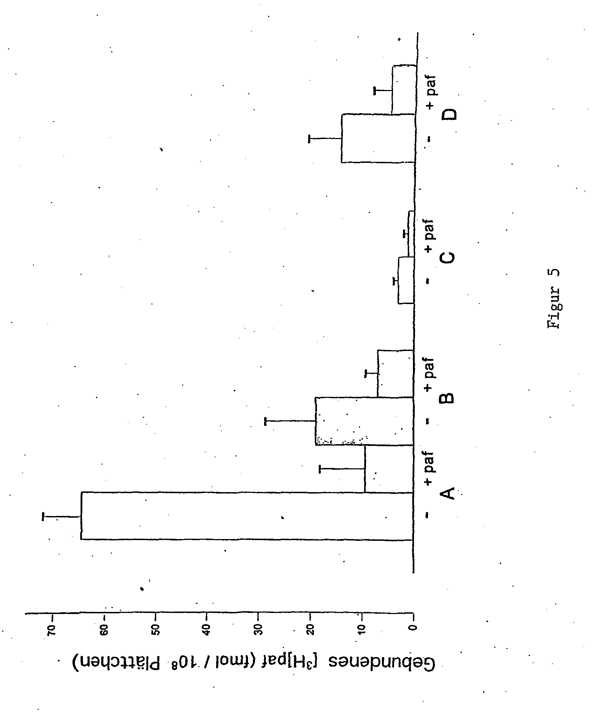

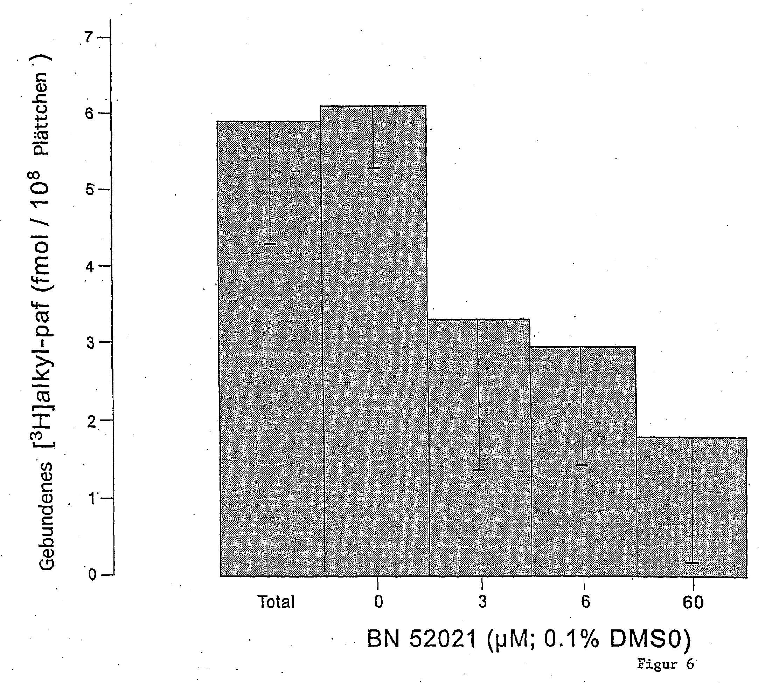

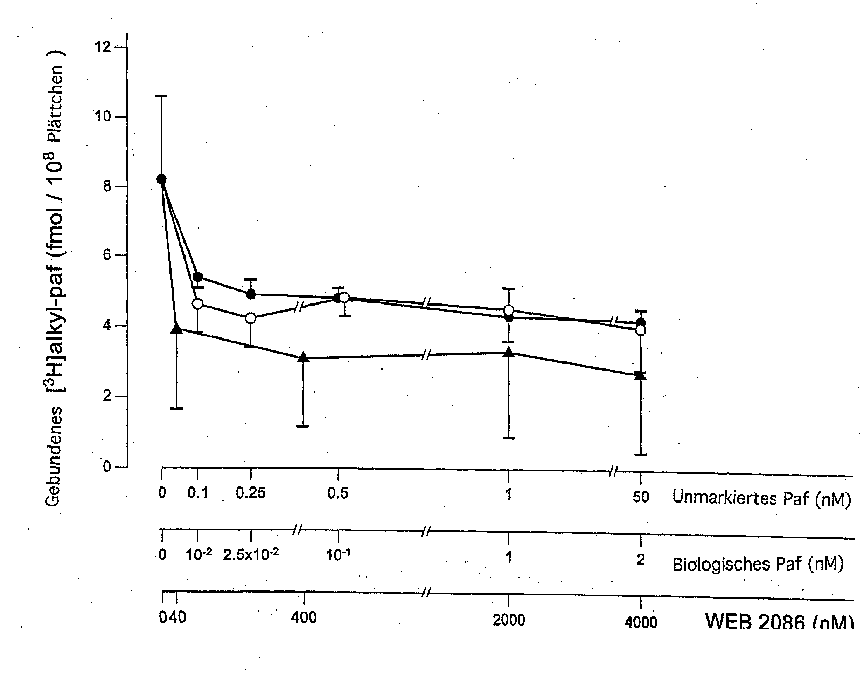

Die Figur 5 zeigt fixierte gewaschene Thrombozyten, die [³H]alkyl paf bei 20 ° C binden. Die Figuren 6 und 7 zeigen, dass Ginkgoloide in konzentrationsabhängiger Weise die Bindung von [³H]alkyl paf an fixierte Thrombozyten hemmen, wobei die klassischen Leitsubstanzen als Standard verwendet werden d.h. BN52021 für die originalen "chemically defined extracts of Ginkgo biloba" und WEB 2086 als Beispiel für die bekannten Chlorophenylbenzo(dia)zepine.

Die Figur 5 zeigt, dass fixierte Zellen markierte Alkyl-Lipide nur bei 20 ° C binden, aber nicht bei 4 ° C (Figur 5 D versus 5C). Der allosterische Alkyl-Effekte wird durch Erstarrung der Membran gehemmt und damit die vorgenannte fressähnliche, allosterische Konfigurationsänderung der Zellen gezeigt. Die [³H]Acetyl Gruppe vom Paf bindet dagegen bei 4 ° C an fixierte Zellen, die vor dem letzten Waschgang mit Formaldehyd behandelt und sodann gekühlt, erstarrt wurden (1%, 30 Min, A+B). Die Acetylgruppe bindet, besetzt somit die Paf Rezeptoren, ohne allosterische Aufregulationen, wobei die acetylierende Substanzen diesen Effekt erfindungsgemäß nützen.

Die Figur 6 zeigt das Auswahlverfahren von Ginkgoloiden mittels fixierten Zellen, fixierten Zellinien, BN 52021 wird als Leitsubstanz der Ginkgolide getestet und hemmt hier die Bindung von [³H]alkyl paf an fixierte Zellen in dosisabhängiger Weise.

Die Figur 7 zeigt erstmals die Bindung einer unaufgetrennten Alykl-Liganden-Mischung aus dem Vollblut ("biologisches paf") an fixierte Thrombozyten. Ein Extrakt aus dem Vollblut d.h. "biologisches Paf" verdrängt [³H]alkyl paf aus seiner Bindung und bindet sich an gleiche Alkyl-Rezeptoren. Diese kompetitive Hemmung verläuft parallel mit synthetischem Paf und mit Ginkgoloiden, wobei hier WEB 2086 als die bekannte Leitsubstanz für Chlorophenylbenzodiazepine getestet wird

"Biologisches Paf" wurde hier erstmals verwendet als unaufgetrennter äthanolischer Extrakt vom Vollblut (ohne HPLC). Dieser gelöste Trockenextrakt wird mit Kaninchenthrombozyten über die Aggregation in Gegenwart von Aspirin und CP/CPK quantifiziert. Die neuen Methoden erlauben erstmals auch Testungen ohne Albumin, so dass Alkyl-Lipide in messbarer Weise binden können mit niedrigerer Affinität als Albumin. Zum Beispiel binden Alkyl-Lipide ohne die Cholingruppe (Alkyl-LPA) sonst in den tieferen Membranregionen von unbehandelten Zellen und/oder werden schnell internalisiert vom freien Albumin. Die Methode ist zudem calciumunabhängig, so dass hier auch calciumunabhängige Alkyl-Lipide (z.B. Lyso paf) gehemmt werden. Der klinische Erfolg der Kompositionen gegen alle Alkyl-Lipide ist somit nachvollziehbar gestützt.

Die Figuren 5 bis 7 zeigen also das neuen Screeningverfahren, das ein neues technisches Problem löste nämlich die Vielfalt der schädlichen Alkyl-Lipide. Mit fixierten Zellen, Zelllinien wird auch die Quantifizierung vereinfacht, denn aufwendigen Reacetylierungen von Alkyl-Lipiden für calciumabhängige Quantifizierungen entfallen. Mit fixierten Zellen, Zellinien können auch markierte Antikörper und/oder lösliche Transport-, Bindungsproteine getestet und hergestellt werden.

### 7. Klinische Beispiele;

Die erfolgreiche klinische Anwendung der neuen Komposition wurde hier erstmals beschrieben. Übergewicht, Fehlernährung mit Butdruckerhöhung, Diabetesneigung, kritische Morgenurine ("OMIH"-Syndrom) wurden geheilt auch nach einem alkoholbedingten hepatorenale Syndrom ("AHA"-Syndrom), wobei die langen Genesungsperioden auch eine genetische Regeneration anzeigte.

### 7.1. Beispiel 1:

Die Tabelle 1 zeigt die erfolgreiche Therapie vom "Rosenkavalier", wobei sein auswärts diagnostiziertes Alkoholsyndrom ("AHA"-Syndrom) hier 2002/2004 geheilt wurde. Sodann entwickelte er ein "OMIH"-Syndrom. Nephrose, Albuminwerte, Thrombozytose, Blutungsrisiko (Ösophagusvarizen) wurden 2003/2004 erfolgreich behandelt (Quick 100%). Die Hypertonie wurde weiter mit β-Blockern behandelt nachdem Diuretika abgesetzt werden konnten. Zunächst wurde ihm die Vollmilch geschenkt, die mit Vitaminen der B-Reihe angereichert wurde, wobei Hafer und Früchte beigemischt wurden (d.h. Eisen, Magnesium in Erdbeeren, Himbeeren) auch als Modell für Patienten in letzter Not (z.B. Hospize, Dialysen).

Ab 2002 erhielt der "Rosenkavalier" die vorgenannte erfindungsgemäße Komposition (FIDA^{R}, Figur 1) auch gegen sein überraschendes "OMIH"-Syndrom der Jahre 2005/2006, das hier auch erfindungsgemäß erfolgreich behandelt wurde. Das Übergewicht blieb, aber die gemischte Hyperlipidämie konnte mit der Komposition ausgeglichen werden (Tabelle 1). Lipidsenker wurden nicht verabreicht im Hinblick auf die Leberzirrhose und auch im Hinblick auf seine gutartigen Isoformen von Lipoproteinen (Apo E Allelen 3/3, kein Allel E4). Die Plasma-Albuminwerte wurden normal, so dass auch die Gefahr von hepatischen und/oder renalen Encephalopathien (sekundäre Demenzen) überwunden war. Eine seelisch, mentale Ausgeglichenheit wurde mit der erfindungsgemäßen Komposition erreicht; die hier belegt ist durch eine fünfjährige Abstinenz ohne Hilfe Dritter und auch mit den Labordaten (Tabelle 1).

Die Hämaturie heilte nachhaltig aus und die Albuminurie besserte sich unter der Behandlung. Der renale Calciumverlust sank von 38 mg/l auf 18 mg/dl pro Liter Urin und Lyso paf sank von 52 pg/l auf 25 pg/l Urin durch die Besserung der Albuminurie. Diese berechneten Werte zeigen den calciumsparenden, zelischonenden Nutzen der erfindungsgemäßen Komposition, wobei in den Jahren 2005/2006 das "OMIH"-Syndrom erfolgreich behandelt werden konnte (Tabelle 1). Die renale Anämie blieb und wird mit Erythropoeitin ausgeglichen.

Die jährliche Oberbauchsonografie bestätigte die Leberzirrhose ohne Karzinogenese. Die kariösen Restzähne vom "Rosenkavalier" könnten behandelt werden, aber nun hat der "Rosenkavalier" ein anderes Leben begonnen im katholischen Männerwohnheim.

Die Untersuchungen der ersten Morgenurine schlossen schwere Nierenschäden aus (z.B 28.10.2005), bestätigten die Mikroalbuminurie (22 mg/g Krea <20) und Proteinurie (129 mg/g Krea <120) aber nun ohne Erythrozyten und pathologische Zylinder in der Urinmikroskopie. Die Tabelle 1 zeigt gute Blutdruckwerte bei bleibender Basistherapie (Propanolol d.h. ß-Blocker und Spironolakton d.h. Aldosteronantagonist). Eine auswärts durchgeführte Messung vom Langzeitblutdruck zeigte einen guten nächtlichen Blutdruckabfall, so dass die renale endotheliale Dysfunktion ausheilen konnte. Die glatte Gefäßmuskulatur blieb sehr empfindlich in den Jahren 2005/2006, denn der Blutdruck stieg anfallsartig bei Belastung an oder ohne die Komposition (z.B. 180/100 mmHg). Das unerwartete "OMIH"-Syndrom der Jahre 2005/2006 war weitgehend ausgeheilt. Eine Überlebenszeit von fünf Jahren nach einer akuten hepatorenalen Insuffizienz überraschte die biomedizinische Fachwelt. Die erfindungsgemäße Komposition zeigte also eine unerwartete Heilung.

Urologische, nephrologische, hepatische, neurovaskuläre, vaskuläre, neurologische, neoplastische, kardiologische zelluläre Störungen, Schlaganfall, Blutungen, sekundäre, vaskuläre Demenzen, Karzinogenese, Nephrosen, hepatische und/oder renale Encephalopathien, und auch die sonst so typischen Knochenbrüche und Wundheilungsstörungen wurden verhindert (bei Z.n. Ulcus cruris). Die schwere alkylbedingte Destabilisierung von Zellen bildete sich zurück, wobei einige Restschäden blieben z.B. die erhöhte Gamma-GT, die renale Anämie, die überempfindliche glatte Gefäßmuskulatur. Die mikrovaskulären Probleme und auch die Suchtprobleme wurden ohne Hilfe Dritter überwunden, denn der "Rosenkavalier" blieb abstinent und nahm die erfindungsgemäßen Kompositionen. Mit der Komposition wurde die körperliche, seelische, geistige, mentale Besserung auch der Lebensführung und des gesamten Organismus erreicht.

### 7.2. Beispiel 2:

Das statistisch signifikante "OMIH"-Syndrom wird in der Tabelle 2 gezeigt, die Voraussagen und klare therapeutische Anweisungen beinhaltet. Die kritische Lebensführung mit ihren additiven schädlichen Effekten ist zu bessern bei den ansonsten noch gesunden Risikopatienten.

Die Tabelle 2 zeigt die signifikanten Assoziationen bei den "OMIH"-Gewichtsproblemen (BMI1+BMI2) mit Blutdruckerhöhung (RR), gemischten Hyperlipidämien (LDL-Trig, Trig.) (p<0.05), LDL-bezogener Glukosetoleranzstörung (LDL-IGTT) besonders bei Alkohol- (AHA, p=0.011) und/oder Nikotinproblemen. Alkoholkonsum war mit steigendem Gewicht und mikrozirkulatorischen Schäden assoziiert (p<0.05). Hormone verursachten keine direkten Schäden.

Die Tabelle 3 zeigt den Verlauf. Abstinente adipöse Frauen zeigen keine renalen Schäden (BMI2) aber einen weiterhin erhöhten Blutdruck, so dass die (Tabelle 3 die blutdrucksteigernde Wirkung vom Fettgewebe bei Adipositas zeigt. Die direkten renalen Schäden bleiben bei Alkoholmissbrauch (AHA), aber der Blutdruck besserte sich mit der Normalisierung der Lipidprofile. Frauen mit Übergewicht (BMI1) waren älter und rauchten häufig (57%) gegen ihr Übergewicht. Die klinischen Verlaufsstudien sind im Prioritätsdokument P3466914 ausführlicher beschrieben und werden hier zitierend einbezogen. Bei kritischen Morgenurinen besonders bei Nikotin- und/oder AlkoholProblemen werden Infektionen und/oder Karzinogenese ausgeschlossen (Streifentests, Proteinprofile, Urinmikroskopie, markierte Proteine, Lipide, Alkyl-Lipide, Matrixprotein, Proteome usw.). Alkyl-Lipide in entarteten Zellen, bei der Karzinogenese werden diagnostisch genützt z.B. gegen urologisch, nephrologische Neoplasien auch mit bildgebenden Verfahren (z.B. im NMR, PET-Scan usw).

### 7.3. Beispiel 3:

Die Tabelle 4 zeigt den Heilungsverlauf einer Hämaturie mit Diabetesneigung als Ausdruck einer Überempfindlichkeit gegenüber Hormonen. Hier wurde eine Hypothyreose durchgehend mit Thyroxin und Jodid eingestellt, wobei sich die Hämaturie mit Diabetesneigung nicht besserte. Erst nach seelisch, geistiger Ausgeglichenheit d.h. verbesserter Lebensführung mit vermindertem Nikotinkonsum (von 20 auf 6 Zigaretten) besserten sich Hämaturie und Prädiabetes nachhaltig, denn die alkylartig Destabilisierung von Zellen wurde gehemmt.

Übergewicht, Blutdruckerhöhungen, kritische Lipidprofile, Albuminurie, Proteinurie, Hämaturie, Nikotinprobleme wurden erfindungsgemäß behandelt auch mit hormonellen Kompositionen. Die Schönheit und Vitalität wird gefördert, das Bindegewebe gestärkt. Seelisch, mentale Probleme konnten gelöst und eine kritische Lebensführung verbessert werden ohne Hilfe Dritter, so dass hier eine hormonelle Therapie gut vertragen werden konnte.

### 8) Vorpublizierter Stand der Technik:

Der Recherchenbericht des Deutschen Patentamts vom 28.9.2006 (Nr.102005062417,0) hält dem P34669914 (Anm. 27.12.2005) die nachgenannten validen US-Patente der Anmelderin entgegen, die in der veröffentlichten US Patentanmeldung No. 2002/0127287 zusammenfassend publiziert sind (Publ. 12.9.2002). Das Gebrauchsmuster DE 29700734 U1 der Anmelderin und das andersseitige OS DE3929763 werden hier ebenfalls zitierend einbezogen.

Keine der Entgegenhaltungen offenbart hormonelle Kompositionen oder legt die beanspruchten Verwendungen nahe. Im Amtbescheid vom 19.6.2003 erkannte das US Patent Office die veröffentlichte US-Patentanmeldung No. 2002/0127287 (Publ. 12.9.2002) als Doppelpatentierung der Mutteranmeldungen an, d.h. von US Patents No. 5,346,894, US-5,605,927 und US No. 5,852,052. Zudem wurden die ebenfalls validen US Patente No. US 5,480,881 und US No. 530.023 entgegengehalten, da Methoden und Verwendungen von/gegen LA-paf offenbart sind und auch mit dem hier zitierend einbezogenen validen EP 0459432. Das zitierte US Patent No. 5696114 wird hier mit EP 0540766 zitierend einbezogen. Das valide US-Patent No. 5,895,785 der Anmelderin wird hier auch zitierend einbezogen. Mit dem Gebrauchsmuster 29700734 U1 (Publ. 26.6.97) offenbarte die Anmelderin viele zeitgerecht originale Mischungen und Verwendungen für lokale und diätetische Darreichungsformen auch mit dem Ginkgo-Spezialextrakt EGB761 mit Nahrungsbestandteilen, Milchprodukten, ungesättigten Fettsäuren und/oder Vitaminen (ohne Hormone, Mineralien, Spurenelemente).

Viele Verwendungen von Paf Antagonisten und/oder Hemmstoffen gegen Etherphospholipide wurden von der Anmelderin vorbeansprucht gegen mentale und zerebrale Erkrankungen, Störungen der Bluthirnschranke, sklerotische Hautprobleme, metabolische Syndrome und suchtartige Erkrankungen mit den zeitgerecht originalen Methoden, Pathomechanismen d.h. Bindung und Umsetzung vom Ether-(Alkyl)-Phospholipiden vorzugsweise Lyso paf und/oder LA-paf. Die zeitgerecht originalen Mischungen von Paf Antagonisten (Ginkgoloiden) z.B. Triazolothienodiazepinen, Ginkgoliden, Ginkgoloiden mit oder ohne Fischöle, Vitamine, Antioxidantien, Steroide, cAMP-Modulatoren, Albumin, Prostaglandin Antagonisten sind mit oralen, diätetischen, lokalen, kosmetischen Darreichungsformen, Liposomen vorbeansprucht und auch für Nahrungsmittel, Nahrungsmittelbestandteile, Schönheitsmittel (Kits).

Folgend werden die validen Europäischen Patente diskutiert, die Prioritäten der Entgegenhaltungen sind. Paf Antagonisten mit Knoblauchölen, Glukosteroiden, Vitaminen und/oder Fettsäuren sind mit EP 540766 offenbart (Publ. 12.5.93). Im EP 0540766 oder EP 0459432 wurden der Schutz der Thrombozyten mit Albumin offenbart und auch die Differenzierung von monozytären Zellen durch Cholesterol und LDL mit Modulation vom cAMP. Außerdem wurden die gesättigten Kinetiken der zellulären Acetylhydrolasen (Phospholipasen) im EP 0540766 offenbart, sowie die gesteigerte Synthese der Acetylhydrolasen durch LD. Das valide EP 0459432 (Publ. 4.12.91) schützt u.a. Paf Antagonisten bei/gegen lipoproteinbedingte Erkrankungen einschließlich der Aggregation von Thrombozyten mit LDL und LA-paf. (DE 4034090, Publ. 30.4.1992; Korth et al. Chem. Phys. Lipids 70, S.109,1994).

Die erfolgreiche Behandlung vom Metabolischen Syndrom d.h. vom Hyperinsulinismus mit Paf Antagonisten basiert auf der Differenzierung von Endothelzellen durch Insulin (EP 0 604830, DE4244265, Publ. 6.7.94; Korth et al. Biochem. Pharmacol. 49, 1793, 1995). Metabolische Syndrome mit LA-paf und Intimaverdickung bei Grenzwerthypertonie sind sodann mit dem Paf Antagonisten WEB 2086 erfolgreich behandelt worden (Wu et al. J. Int. Med. 246, S.389, 1999).

Der Schutz mit Gingkoloiden ist offenbart für endotheliale Barrieren bei metabolischen Syndromen und/oder bei alkoholbedingten Hyperlipidämien (R.-M. Korth, Rec. Res. Devel. Lipids 5, S. 61, 2001; Journal of men's health & gender, vol 3, S. 279-289, 2006). Die klinischen Studien bestätigen hier die vorgenannten Schutzrechte, die zitierend einbezogen sind. Die vorliegende Erfindung überraschte aber klinisch mit neuen Syndromen, den Überempfindlichkeitssyndromen und/oder dem unerkannten Calciumverlust, den Pigmentstörungen usw.

Mit dem validen EP 0540767 (Publ. 12.5.1993) offenbarte die Anmelderin erstmals die mentalen und zerebralen Erkrankungen, die von einem erhöhten Gehalt von Lyso paf begleitet sind ("Lyso paf-Syndrome") und zeigte erstmals einen klaren Pathomechanismus für Psychosen u.a. bei der SDAT. Lyso paf bindet sich an spezifische Rezeptoren mit aufregulierender Wirkung, wobei die Proteinkinasen C (PKC, PMA) auch die Wirkung anderer zerebraler Botenstoffe verstärken (EP 0540767. Publ. 12.5.93). Paf Antagonisten (auch EGB761 usw) begegnen erstmals mentalen und zerebralen Krankheiten durch eine Hemmung von aufregulierenden Lyso paf Rezeptoren, so dass deren Verwendungen vorbeansprucht wurden im validen EP 0540767 für Psychosen, wahnhaften, affektiven, sensitiven, disharmonischen, nebensinnigen Störungen, Affekt-, Charakterstörungen, erlebnisreaktiven Störungen und bei mentalen Grenzwert (borderline) Syndromen, die heute als paranoide Störungen zusammengefasst werden. Das jugendliche (hebe) Gehirn (Phrenia) ist dabei besonders gefährdet. EP 0540767 legt keine renalen Schäden durch alkylartigen Lipide nahe.

Die Anmelderin offenbart, beanspruchte "Aufarbeitungen von Ginkgoliden als Gemisch" im EP 0312913 (Publ. 26.4.89) zum Endothelschutz. Das valide EP 0312913 (Publ. 26.4.89) offenbarte auch EGB 761 und dessen perkutane Verwendungen. Auch Paf Analoge wurden offenbart (R. Korth et al., Chem. Phys. Lipids 36, S. 209, 1985, R. Korth et al., Eur. J. Pharmacol. 152, S. 101, 1988, R. Korth, Vascular endothelium Nato Asi, Plenum Press, eds. Catravas et al., S.89-98 1989). Die Rezeptorhemmung von Paf Antagonisten ist hochwertig publiziert mit den biologisch aktiven Ether-Phospho(ryl)cholinen PAF-Acether, LA-paf oder Lyso paf (R.-M. Korth: EP 0312913, EP 0540767, Korth et al. Eur. J. Pharmacol. 152, 101-110, 1988; Chem.Phys. Lipids, 70, S.109, 1994; R.-M. Korth et al., Biochem. Pharmacol. 49, S. 1793,1995; R.-M. Korth, Int. Arch. All. Imm. 113, S. 460, 1997; Meade et al., Biochem. Pharmacol., 41, S.657-668, 1991; Hwang et al., Biochim. Biophys. Acta 1085, 91-105, 1991).

Dagegen führten hier auch neue Screeningverfahren zu überraschenden Komponenten z.B. mit acetylierenden Substanzen und zu neuen Kompositionen. Neue Screeningmethoden werden hier erstmals spezifiziert zur Auswahl von neuen Kompositionen und/oder neuen Komponenten, um unerwartete AAGPC mit seinen Derivaten, Produkten zu hemmen (z.B. Alkyl-Glycerole, Alkyl-LPA, Cannboide, Acetylacyl-CoA usw.).

Der instabile Entzündungsmediator PAF ist bekanntlich ein hypotensives Phospholipid d.h. ein negativ inotroper Mediator und damit führt das entgegengehaltene OS DE3929763 von der vorliegenden Erfindung weg. Im zitierten OS DE3929763 (Publ. 14.3.91) werden Paf-Antagonisten (mit Magnesium) verwendet bei "zu geringen Pumpleistungen des Herzens... Herzinsuffizienz. zur Behandlung der durch verminderte ß-Rezeptorenstimulation am Herzen", eben weil PAF negativ inotrop ist (OS DE 3929763, S. 2, Z.18-42). PAF ist negativ inotrop und somit hypotensiv und deshalb sollen die Paf Antagonisten andersseitig positiv inotrop wirken. Bei der Herzinsuffizienz soll der Blutdruck gesteigert werden wogegen der Blutdruck hier erfindungsgemäß gesenkt wird z.B. bei den Überempfindlichkeitssyndromen. Zudem ist hier ein synergistischer, antihypertensiver aber kein antagonistischer hypertensiver Effekt mit ß-Rezeptorenblockern erwünscht. Viele Antidementiva (z.B. Cholinesterasehemmer) können nämlich nicht mit ß-Blockern kombiniert werden, so dass hier eine wichtige Differentialtherapie für Ginkgoloide erstmals offenbart wird für ältere, herzgesunde Patienten mit ordentlich behandeltem Hochdruck.

Die hormonelle Komposition ist neu und überrascht. Keine der nachpublizierten Schutzrechte Dritter legte die Komposition nahe und/oder deren Verwendungen. Die Ansprüche Dritter bleiben in jeder Hinsicht hinter dem vorliegenden Erfindungsgegenstand zurück. Man reinigt die gereinigten Extrakte (vgl. EP 360556, Anm. 19.9.89 mit EP 1037646, Anm. 19.12.1997), aber befreit die Extrakte z.B. nicht von ätherischen Ölen und zudem werden keine antioxidativ stabilisierten Öle verwendet. Neuheitlich begründet wird andersseitig mit der Reihenfolgen, von ansonsten eher handwerklichen Verfahrensschritten, um einige Ginkgolidprozente mehr zu gewinnen in den "Spezialextrakten" vom Ginkgo biloba (product by process: Reinigung/limitiation usw.). Die vorbeanspruchten späten Durchblutungsstörungen, hirnorganische Störungen und/oder manifeste Hirnleistungsstörungen bieten keine Hinweise, Anweisungen für ansonsten gesunde Risikopersonen. Diese Herstellungsverfahren sind hier auch nicht entscheidend, weil die neuen Screeningverfahren hier aus einer Vielzahl von Extrakten auswählen (EGB, EGB761 usw). Zudem werden neue Herstellungsverfahren im Prioritätsdokument P34669914 ausführlich spezifiziert, die besser geeignet sind für die öligen, kostengünstigeren Aufarbeitungen der neuen diätetischen Kompositionen oder die neuen lokalen Kompositionen ohne ätherische Öle.

Synthetische Substanzen wie z.B. Cholorphenyl-Ether-Benzozepinderivate finden hier eine neue zweite Verwendung gegen AAGPC seine Derivate, Produkte, denn der alkylacylartige Zellschaden wird hier durch die additiven Schäden bei Konsum von Alkohol und/oder Nikotin klinisch belegt und erstmals behandelt. Die vorveröffentlichten Risikofaktoren z.B. erhöhte Werte vom LDL, Cholesterol und/oder Triglyceriden (z.B. im EP 751774) sind hier nicht signifikant und verursachen noch kein frühes "OMIH"-Syndrom.

Mit Nahrungstabellen und Selbstkontrolldokumentationen wird Nahrung empfohlen (nicht beansprucht) z.B. Fisch, Kartoffeln, fettarme Milch-, Molkeprodukte, Kohlsorten, Fenchel, Lauch, Beeren, Gewürze, Kräuter und die calciumreiche Petersilie. Besonders viele Prähormone sind z.B. in Brunnenkresse enthalten (Herba Nasturtii, Phytosterine, Alkaloide, Aminosäuren). Milch- Molkeprodukte enthalten bekanntlich alle lichtabhängigen Hormone. Das Albumin trägt dabei ungefähr 1.2 g/l Calcium besonders für Kinder, Jugendliche während des Knochenwachstums. Fettarme Milch-, Molkeprodukte werden z.B. mit Hafer ergänzt mit seinem hohen Gehalt an Aminosäuren, Vitaminen vorzugsweise Vitamin K und E und Provitamin A (Carotin), Mineralstoffen wie Phosphor, Eisen, Kobalt, Zink, Aluminium, Kalium und den Spurenelementen Bor, Selen und Jod. Zellulose, Getreideprodukte mit Selen sind bekannt, um die Rückresorption von Fetten zu verhindern. Auch Flavonoide, Knoblauchprodukte (Allium sativum) sind weitgehend freier Stand der Technik sowie Phyto-Hormone, Vitamine, Allicin, Cholin, Jod und viele Lipidsenker. Genetische Risiken, Rauchen, Alkohol, Kaffee sind die bekannten Gründe für Hypovitaminosen, wobei auch das freie Vitamin D hier zitierend einbezogen ist (Jacobus et al., N Engl J Med 1992, 326,1173-7). Der freie Stand der Technik z.B. Vitamin D in Milchprodukte ist zitierend einbezogen (Holick et al, N Engl J Med 1992; 326, 1178-81). Milch enthält bekantlich viele Peptide (z.B. Albumin) und Enzyme (z.B. Biotine).

Hier wurde erstmals ein seelisch geistiger Ausgleich vom Menschen mit Besserung der Lebensführung belegt. Erstmals wird eine nachhaltige Heilung von seelisch, geistigen, körperlichen Schäden gezeigt. Auch ein Calciumverlust bei/durch Albuminurie wird hier erstmals erkannt, behandelt.

Auch neuere Ballaststoffe (z.B. Plantago ovata) werden den neuen Kompositionen beigemischt gegen das überraschende "OMIH"-Syndrom. Die neuen lokalen und/oder diätetischen Kompositionen schützten erstmals gegen Alkyl-Acyl-GPC, seine Derivate, Produkte gegen zelluläre Überempfindlichkeitssyndrome und Störungen der Zellen im gesamten Organismus (z.B. AAGPC, AcetylAcyl-CoA, Alkyl-LPA, Alkyl-Cannaboide, Alkyl-Ethanolamine usw). Neue Kompositionen wurden klinisch erarbeitet, die aus gereinigten, aufgearbeiteten, stabilisierten und/oder semi-synthetischen Komponenten bestehen. Die Kompositionen enthalten erstmals auch eine Mischung aus Ginkgoloiden mit Calcium (oder Phosphor), wogegen bisher nur Antagonisten gegen das Calcium als Antihypertensiva und Antidementiva verwendet wurden, die vom Ausgleich des Calcium- und Phosphatverlusts wegführten.

Die seelisch geistige Verfassung des Menschen wird hier erstmals durch die erfindungsgemäßen Kompositionen auch hormonell und mit der Lebensführung verbessert. Erstmals wirken Ginkgoloide, Hormone, Prähormone, Mineralien, Spurenelemente und/oder Acetylcysteine gegen die unerwartetes Übergewichts-, Überempfindlichkeitssyndrome und/oder Pigmentstörungen, wobei der jahrelange pflegende Therapieverlauf hier erstmals die (genetische) Regeneration aller Zellen im gesamten Organismus belegt.

## Patentansprüche

1. Eine hormonelle Komposition, **gekennzeichnet durch** zumindest ein
a) Prähormon / Hormon aus der Mevalonat-, Cholecalciferol-Calcitriolgruppe, und Ergocalciferolgruppe, und
b) Ginkgoloid gegen Alkyl-Acyl-GPC, ausgewählt aus der Gruppe der Ginkgolide, und
c) ein Mineral aus der Phosphor- und Calciumgruppe, und
d) ein Spurenelement.

2. Eine ölige Komposition nach Anspruch 1, **dadurch gekennzeichnet, daß** die Öle antioxidativ stabilisiert sind.

3. Eine Komposition mit Acetylcystein, **dadurch gekennzeichnet, daß** Acetylcystein mit zumindest einem Ginkgolid kombiniert ist.

4. Eine Komposition der Ansprüche 1 bis 3 zur Verwendung bei der Behandlung eines Übergewichtssyndroms mit Hyperlipidämie und/oder Glukoseintoleranz, Bluthochdruck und kritischen Morgenurinen.

## Claims

1. A hormonal composition **characterized by** at least one
a) prehormone/hormone from the group of mevalonates, the group of cholecalciferol-calcitriol and the group of ergocalciferol, and
b) Ginkgoloid against alkyl-Acyl-GPC, selected out of the group consisting of Ginkgolides, and
c) a mineral from the group of phosphor, the group of calcium, and
d) a trace element

2. An oily composition of claim 1, wherein the oils comprise an antioxidant for stabilization.

3. A composition with acetylcysteine, wherein the acetylcysteine is combined with at least on ginkgolide.

4. A composition in accordance with claims 1 to 3 used for treatment in cases with overweight syndrom, **characterized by** hyperlipidemia and/or intolerance to glucose, elevated blood pressure and critical morning urines.

## Revendications

1. Une composition hormonelle, characterisée comprenant au moins
a) une préhormone/hormone issu des groupes des mévalonates, des cholecalciferoles/calcitrioles et de la groupe d' ergocalciferoles, et
b) un ginkgoloide pour lutter contre le alkyle-acyl-GPC selectioné de la groupe des ginkgolides, et
c) un minéral issu de la groupe des phosphor et calcium, et
d) un oligo-élément.

2. Une huileuse composition selon la revendication 1, characterisée en ce que les huiles sont stabilisées avec des antioxidants.

3. Une composition comprenant de l'acétylcysteine, characterisée en ce que l'acétylcysteine est combinée avec au moins un ginkgolide.

4. Une composition selon des revendications 1 à 3 pour usage dans le cas de traitement d' une syndrome characterisée par surcharge pondérale avec l'hyperlipidemie et/ou intolerance au glucose, hypertension et des urines du matin critiques.
